(19) Europäisches Patentamt / European Patent Office / Office européen des brevets

(11) EP 4 778 930 A1

(12) EUROPEAN PATENT APPLICATION
published in accordance with Art. 153(4) EPC

(43) Date of publication:
**22.07.2026 Bulletin 2026/30**

(21) Application number: **24864722.4**

(22) Date of filing: **13.09.2024**

(51) International Patent Classification (IPC):
***C07D 493/04*** (2006.01) ***A01N 25/02*** (2006.01)
***A01N 53/00*** (2006.01) ***A01N 53/04*** (2006.01)
***A01P 7/00*** (2006.01) ***A01P 7/02*** (2006.01)
***A01P 7/04*** (2006.01)

(52) Cooperative Patent Classification (CPC):
**A01N 25/02; A01N 43/90; A01N 53/00; A01P 7/00; A01P 7/02; A01P 7/04; C07D 493/04**

(86) International application number:
**PCT/CN2024/118653**

(87) International publication number:
**WO 2025/056008 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.09.2023 CN 202311189770**
**19.12.2023 CN 202311747118**
**09.08.2024 CN 202411096509**

(71) Applicant: **Qingdao KingAgroot Chemical Compound Co., Ltd.**
**Qingdao, Shandong 266000 (CN)**

(72) Inventors:
- **LIAN, Lei**
  **Qingdao, Shandong 266000 (CN)**
- **PENG, Xuegang**
  **Qingdao, Shandong 266000 (CN)**
- **HUA, Rongbao**
  **Qingdao, Shandong 266000 (CN)**

(74) Representative: **Zanoli, Enrico et al**
**Zanoli & Giavarini S.p.A.**
**Via Melchiorre Gioia, 64**
**20125 Milano (IT)**

# (54) PYRIPYROPENE DERIVATIVE, AND COMPOSITION AND USE THEREOF

(57) The present invention belongs to the technical field of pesticides, and particularly relates to a pyripyropene derivative, and a composition and the use thereof. The compound is as shown by general formula I,

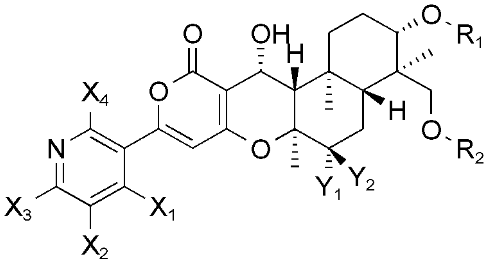

I

wherein, $X_1$ represents hydrogen or fluorine; $X_2$ represents hydrogen or fluorine; $X_3$ represents hydrogen or fluorine; $X_4$ represents hydrogen or fluorine; and $X_1$, $X_2$, $X_3$ and $X_4$ are not hydrogen simultaneously; $R_1$ and $R_2$ each independently represent hydrogen, alkyl, alkenyl, alkynyl, cycloalkyl, cycloalkylalkyl, or -(CO)$R_3$; $Y_1$ represents hydroxyl, alkoxyalkyloxy, trialkylsilyloxy, or -O(CO)$R_3$; $Y_2$ represents hydrogen or alkyl; or $Y_1$ and $Y_2$ together form =O. The compound has an excellent prevention and control effect on pests such as *Myzus persicae, Apolygus lucorum, Rhopalosiphum padi* and *Bemisia tabaci.*

EP 4 778 930 A1

## Description

Technical Field

The present invention belongs to the technical field of pesticides and particularly relates to a pyripyropene derivative, a composition and use thereof.

Background Art

To date, a great number of harmful organism control agents with insecticidal activity have been reported. However, insect species that are resistant to these harmful organism control agents or are difficult to control with these harmful organism control agents have been found, and there are further safety concerns for human and animals. Therefore, it is still expected that new harmful organism control agents with strong insecticidal activity will be developed.

Contents of the Invention

In order to solve the above-mentioned problems existing in the prior art, the present invention provides a pyripyropene derivative, a composition and use thereof. The compound has excellent control effects on insect pests such as *Myzus persicae, Apolygus lucorum, Rhopalosiphum padi, Bemisia tabaci,* etc.

The technical solution adopted in the present invention is as follows:
A pyripyropene derivative is as shown in Formula I,

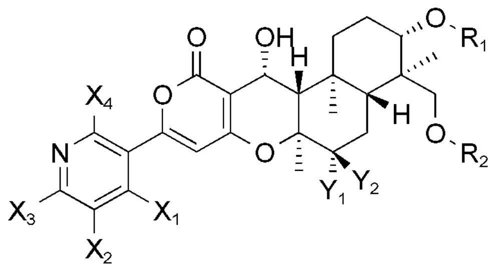

I

wherein, $X_1$ represents hydrogen or fluorine; $X_2$ represents hydrogen or fluorine; $X_3$ represents hydrogen or fluorine; $X_4$ represents hydrogen or fluorine; and $X_1$, $X_2$, $X_3$ and $X_4$ are not simultaneously hydrogen;

$R_1$ and $R_2$ each independently represent hydrogen, alkyl, alkenyl, alkynyl, cycloalkyl, cycloalkylalkyl or -(CO)$R_3$;

$Y_1$ represents hydroxyl, alkoxyalkyloxy, trialkylsilyloxy, or -O(CO)$R_3$; $Y_2$ represents hydrogen or alkyl; or $Y_1$ and $Y_2$ together form =O;

$R_3$ each independently represents hydrogen; alkyl, alkenyl or alkynyl that is unsubstituted or substituted by at least one group selected from halogen, alkoxy, alkoxycarbonyl and alkylthio; cycloalkyl; cycloalkenyl; aryl; or heterocyclyl;

the aforementioned "cycloalkyl", "cycloalkenyl", "heterocyclyl" or "aryl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -$OR_{10}$, -$SR_{10}$, -(CO)$OR_{10}$, -($SO_2$)$R_{10}$, - N($R_{10}$)$_2$ and -O-alkylene-(CO)$OR_{10}$, or two adjacent carbon atoms on the ring form a fused ring with unsubstituted or halogen-substituted -$OCH_2CH_2$- or -$OCH_2O$-;

$R_{10}$ each independently represents hydrogen, alkyl, haloalkyl, phenyl, or phenyl that is substituted by at least one group selected from halogen, cyano, nitro, alkyl, haloalkyl, alkoxycarbonyl, alkylthio, alkylsulfonyl, alkoxy and haloalkoxy.

In one specific embodiment,

$R_1$ and $R_2$ each independently represent hydrogen, C1-C8 alkyl, C2-C8 alkenyl, C2-C8 alkynyl, C3-C8 cycloalkyl, C3-C8 cycloalkyl C1-C8 alkyl or -(CO)$R_3$;

$Y_1$ represents hydroxyl, C1-C8 alkoxy C1-C8 alkyloxy, tri C1-C8 alkylsilyloxy, or -O(CO)$R_3$; $Y_2$ represents hydrogen or C1-C8 alkyl; or $Y_1$ and $Y_2$ together form =O;

$R_3$ each independently represents hydrogen; C1-C8 alkyl, C2-C8 alkenyl or C2-C8 alkynyl that is unsubstituted or substituted by at least one group selected from halogen, C1-C8 alkoxy, C1-C8 alkoxycarbonyl and C1-C8 alkylthio; C3-C8 cycloalkyl; C3-C8 cycloalkenyl; aryl; or heterocyclyl;

the aforementioned "C3-C8 cycloalkyl", "C3-C8 cycloalkenyl", "heterocyclyl" or "aryl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -$OR_{10}$, -$SR_{10}$, -(CO)$OR_{10}$, -$(SO_2)R_{10}$, -$N(R_{10})_2$ and -O-(C1-C8 alkylene)-(CO)$OR_{10}$, or two adjacent carbon atoms on the ring form a fused ring with unsubstituted or halogen-substituted -$OCH_2CH_2$- or -$OCH_2O$-;

$R_{10}$ each independently represents hydrogen, C1-C8 alkyl, halo C1-C8 alkyl, phenyl, or phenyl that is substituted by at least one group selected from halogen, cyano, nitro, C1-C8 alkyl, halo C1-C8 alkyl, C1-C8 alkoxycarbonyl, C1-C8 alkylthio, C1-C8 alkylsulfonyl, C1-C8 alkoxy and halo C1-C8 alkoxy.

In one specific embodiment,

$R_1$ and $R_2$ each independently represent hydrogen, C1-C6 alkyl, C2-C6 alkenyl, C2-C6 alkynyl, C3-C6 cycloalkyl, C3-C6 cycloalkyl C1-C6 alkyl or -(CO)$R_3$;

$Y_1$ represents hydroxyl, C1-C6 alkoxy C1-C6 alkyloxy, tri C1-C6 alkylsilyloxy, or -O(CO)$R_3$; $Y_2$ represents hydrogen or C1-C6 alkyl; or $Y_1$ and $Y_2$ together form =O;

$R_3$ each independently represents hydrogen; C1-C6 alkyl, C2-C6 alkenyl or C2-C6 alkynyl that is unsubstituted or substituted by at least one group selected from halogen, C1-C6 alkoxy, C1-C6 alkoxycarbonyl and C1-C6 alkylthio; C3-C6 cycloalkyl; C3-C6 cycloalkenyl; aryl; or heterocyclyl;

the aforementioned "C3-C6 cycloalkyl", "C3-C6 cycloalkenyl", "heterocyclyl" or "aryl" is optionally substituted by 1-3 groups selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, -$OR_{10}$, -$SR_{10}$, -(CO)$OR_{10}$, -$(SO_2)R_{10}$, -$N(R_{10})_2$ and -O-(C1-C6 alkylene)-(CO)$OR_{10}$, or two adjacent carbon atoms on the ring form a fused ring with unsubstituted or halogen-substituted -$OCH_2CH_2$- or -$OCH_2O$-;

$R_{10}$ each independently represents hydrogen, C1-C6 alkyl, halo C1-C6 alkyl, phenyl, or phenyl that is substituted by 1-3 groups selected from halogen, cyano, nitro, C1-C6 alkyl, halo C1-C6 alkyl, C1-C6 alkoxycarbonyl, C1-C6 alkylthio, C1-C6 alkylsulfonyl, C1-C6 alkoxy and halo C1-C6 alkoxy.

In one specific embodiment,
$X_1$ represents hydrogen; $X_2$ represents fluorine; $X_3$ represents hydrogen; $X_4$ represents hydrogen; or, $X_1$ represents hydrogen; $X_2$ represents hydrogen; $X_3$ represents fluorine; $X_4$ represents hydrogen.

In one specific embodiment,
$Y_1$ represents hydroxyl or -O(CO)$R_3$; $Y_2$ represents hydrogen; or $Y_1$ and $Y_2$ together form =O.

In the definition of the compound represented by the above-mentioned general formula and all of the following structural formulas, the technical terms used, whether used alone or used in compound words, represent the following substituent groups: an alkyl group which has more than two carbon atoms and may be linear or branched. For example, the alkylene in the compound word "-O-alkylene-(CO)$OR_{10}$" may be - $CH_2$-, -$CH_2CH_2$-, -$CH(CH_3)$-, -$C(CH_3)_2$-, etc. An alkyl group is, for example, C1 alkyl: methyl; C2 alkyl: ethyl; C3 alkyl: propyl such as n-propyl or isopropyl; C4 alkyl: butyl such as n-butyl, isobutyl, tert-butyl, or 2-butyl; C5 alkyl: pentyl such as n-pentyl; C6 alkyl: hexyl such as n-hexyl, isohexyl, and 1,3-dimethylbutyl. Similarly, alkenyl is, for example, vinyl, allyl, 1-methylprop-2-en-1-yl, 2-methylprop-2-en-1-yl, but-2-en-1-yl, but-3-en-1-yl, 1-methylbut-3-en-1-yl, and 1-methylbut-2-en-1-yl. Alkynyl is, for example, ethynyl, propargyl, but-2-yn-1-yl, but-3-yn-1-yl, and 1-methylbut-3-yn-1-yl. A multiple bond may be at any position of each unsaturated group. Cycloalkyl is a saturated carbocyclic ring system having for example three to six carbon atoms such as cyclopropyl, cyclobutyl, cyclopentyl, or cyclohexyl. Similarly, cycloalkenyl is monocyclic alkenyl having for example three to six carbon ring members such as cyclopropenyl, cyclobutenyl, cyclopentenyl, and cyclohexenyl, wherein a double bond can be at any position. A halogen is fluorine, chlorine, bromine, or iodine.

Unless otherwise specified, the "aryl" of the present invention includes, but is not limited to, phenyl, naphthyl,

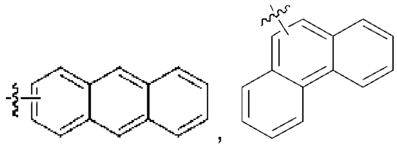
, ;

the "heterocyclyl" not only includes, but is not limited to, saturated or unsaturated non-aromatic cyclic groups

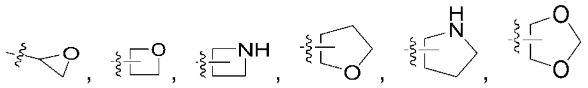
, , , , , ,

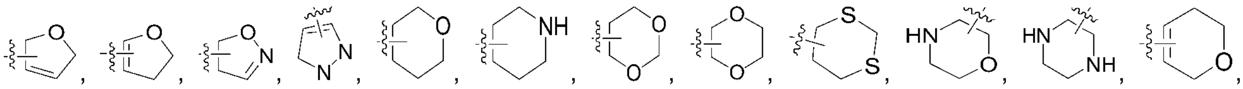
, , , , , , , , , , , , ,

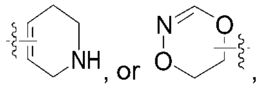
, or ,

etc., but also includes, but is not limited to, heteroaryl, that is an aromatic cyclic group having for example 3 to 6 ring atoms and also optionally fused with a benzo ring. One to four (for example, 1, 2, 3, or 4) heteroatoms of the ring atoms are selected from oxygen, nitrogen, and sulfur, for example

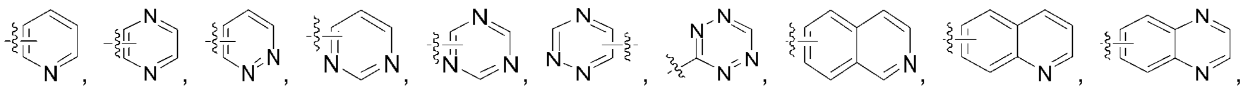
, , , , , , , , , ,

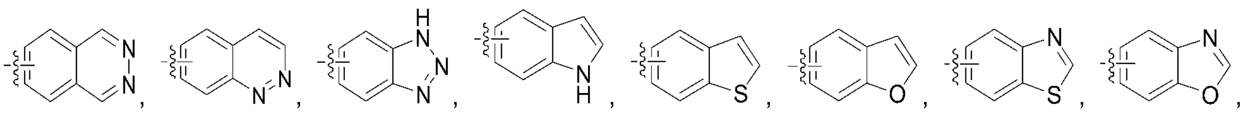
, , , , , , , ,

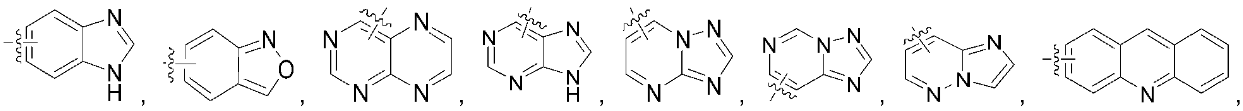
, , , , , , , ,

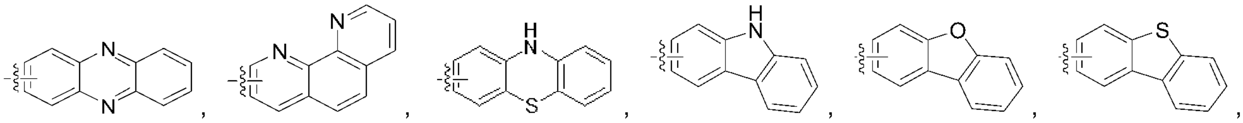
, , , , , ,

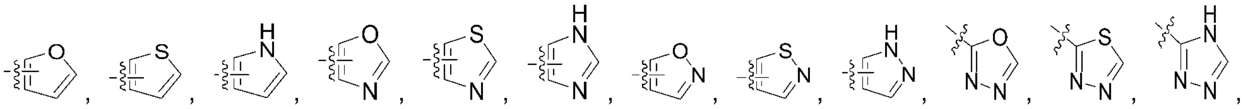
, , , , , , , , , , , ,

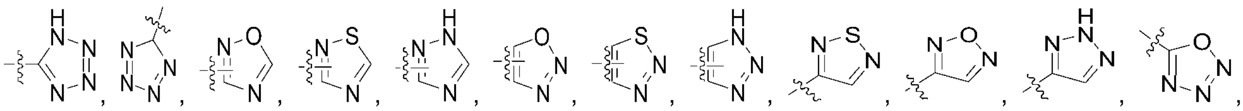
, , , , , , , , , , , ,

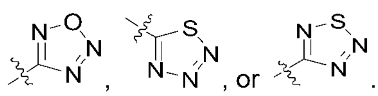
, , or .

If one group is substituted by a group, which should be understood to mean that the group is substituted by one or more the same or different groups selected from the mentioned groups. In addition, the same or different substitution characters contained in the same or different substituent groups are independently selected, which may be the same or different. This is also applicable to a ring system formed by different atoms and units. Meanwhile, the scope of the claims will exclude those compounds chemically unstable under standard conditions which are known to a person skilled in the art.

In addition, unless specifically defined, the term "substituted by at least one group" herein refers to being substituted by for example 1, 2, 3, 4, or 5 groups; a group (including heterocyclyl, aryl, etc.) without being specified a linking site may be linked at any position, including a site connected to C or N; if it is substituted, the substituent group may also substitute at any position as long as the valence bond theory is complied with. For example, the heteroaryl

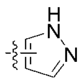

substituted by 1 methyl group may represent

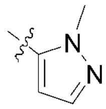,

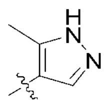, 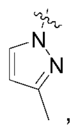, 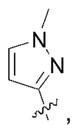,

etc.

A compound represented by Formula I or a salt thereof as an active ingredient has insecticidal effects on pest species including for example lepidopteran pests such as *Spodoptera litura, Mamestra brassicae, Pseudaletia separata,* green caterpillar, *Plutella xylostella, Spodoptera exigua, Chilo suppressalis, Cnaphalocrocis medinalis,* Tortricidae, Carposinidae, Lyonetiidae, Lymantriidae, *Agrotis* spp. pests, *Helicoverpa* spp. pests, or *Heliothis* spp., etc.; hemipteran pests such as Aphididae, Adelgidae, or Phylloxeridae for example *Myzus percicae, Aphis gossypii* Glover, *Aphis fabae, Aphis maidis, Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola,* Rosy apple aphid, *Eriosoma lanigerum, Toxoptera aurantii* or *Toxoptera citricidus,* Deltocephalidae such as *Nephotettix cincticeps,* leafhoppers such as Tea green leafhopper, Delphacidae such as *Laodelphax striatellus, Nilaparvata lugens* or *Sogatella furcifera,* Pentatomidae such as *Eysarcoris ventralis, Nezaraviridula* or *Trigonotylus caelestialium,* Aleyrodidae such as *Bemisia tabaci* or *Trialeurodes vaporariorum,* Coccoidea such as *Pseudococcus comstocki, Planococcus citri* Risso or *Aonidiella aurantii* (e.g., Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, or Cerococcidae), and Psyllidae such as *Diaphorina citri;* Coleoptera pests, for example *Lissorhoptrus oryzophilus, Callosobruchus chinensis, Tenebrio molitor, Diabrotica virgifera virgifera, Diabrotica undecimpunctata howardi, Anomala cuprea, Anomala rufocuprea, Phyllotreta striolata, Aulacophora femoralis, Leptinotarsa decemlineata, Oulema oryzae, Grapholita molesta* or Cerambycidae; Acari, for example *Tetranychus urticae, Tetranychus kanzawai* or *Panonychus citri;* hymenopteran pests, for example Tenthredinoidea; orthopteran pests, for example, Acrididae; dipteran pests, e.g., *Musca domestica* Linnaeus or Agromyzidae; thysanopteran pests, e.g., *Thrips palmi* Karny or *Frankliniella occidentalis;* as well as plant parasitic nematodes, e.g., *Meloidogyne hapla, Pratylenchus, Aphelenchoides besseyi* or *Bursaphelenchus xylophilus.* Examples of animal parasites include Siphonaptera such as *Ctenocephalides felis* or *Pulex irritans;* Anoplura such as *Pediculus* spp. or *Phtirus* spp.; Acari, e.g., *Boophilus* spp., *Haemaphysalis longicornis, Rhipicephalus sanguineus, Haemaphysalis flava, Sarcoptes* spp., *Dermanyssus* spp., *Ornithonyssus sylviarum, Ornithonyssus bacoti,* and *Leptotrombidium;* Tabanidae; dipteran insects such as *Lucilia* spp.; mosquitoes such as *Stegomyia albopicta* and *Culex pipiens pallens;* Simuliidae; Ceratopogonidae; Nematoda, for example *Strongyloides* such as *Strongyloides papillosus* or *Strongyloides stercoralis,* hookworms such as *A. caninum, Ancylostoma tubaeforme* or *Ancylostoma duodenale; Haemonchus* spp.; Strongylida, e.g., mouse Strongyloides; hairworms; Metastrongyloidea, e.g., *Metastrongylus spp., Angiostrongylus cantonensis, or Aelurostrongylus;* Oxyurida; Heterakidae, e.g., *Heterakis gallinarum; Anisakis simplex;* Ascaroidea, e.g., *Ascaris suum, Parascaris equorum, Toxicara canis,* or *Toxocara cati;* Subuluridae; Spiruroidea, e.g., *Gnathostoma spinigerum, Phys aloptera, Ascarops strongylina, Draschia megastoma, Acuaria* spp., or *Ostertagia*

*ostertagi;* Filariidae, e.g., *Dirofilaria* or *Onchocerca cervicalis;* the Order Dioctophymatida; Wipeworms and Trichinosis, e.g., *Trichuris vulpis* or *Trichinella spiralis;* Trematoda, for example Schistosomatoidea such as *Schistosoma japonicum* or *Fasciola hepatica;* Acanthocephala, e.g., *Macracanthorhynchus hirudinaceus* or *Moniliformis moniliformis;* Cestoda, for example, Bothriocephaloidea such as *Diphyllobothrium mansoni;* Cyclophyllidea, e.g., *Dipylidium caninum, Hymenolepis diminuta, Echinococcus multilocularis* or *Echinococcus granulosus;* and protozoa. Preferred pest species include the hemipteran, dipteran and thysanopteran pests. Particularly preferred are the hemipteran pests.

Preferred hemipteran insect pests include Aphididae, Adelgidae or Phylloxeridae (preferably Aphididae); leafhoppers, Aleyrodidae, Pentatomidae or Coccoidea (Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae or Cerococcidae); and Psyllidae, more preferably *Myzus persicae, Aphis gossypii* Glover, Tea green leafhopper, *Bemisia tabaci, Trialeurodes vaporariorum, Trigonotylus caelestialium* or *Pseudococcus comstocki, Aonidiella aurantii* and *Diaphorina citri.*

When a compound represented by Formula I is used as a harmful organism control agent, the compound represented by Formula I can be used directly. Alternatively, a compound represented by Formula I may be mixed with agriculturally or animal husbandry-acceptable carriers such as solid carriers, liquid carriers and gaseous carriers, surfactants, dispersants or other adjuvants for formulation to prepare any suitable formulation, e.g., emulsifiable concentrate, EW (oil-in water emulsion), liquid formulation, suspension, wettable powder, water-dispersible granule, dustable powder, DL dusts, fine granule, granule, tablet, oil, aerosol, floable, dry floable, or microcapsule.

Solid carriers include, for example talc, bentonite, clay, kaolin, diatomaceous earth, vermiculite, white carbon, or calcium carbonate.

Liquid carriers include, for example, alcohols, e.g., methanol, n-hexanol or ethylene glycol; ketones, e.g., acetone, methyl ethyl ketone or cyclohexanone; aliphatic hydrocarbons, e.g., n-hexane, kerosine or kerosene; aromatic hydrocarbons, e.g., toluene, xylene or methylnaphthalene; ethers, e.g., diethyl ether, dioxane or tetrahydrofuran; esters, e.g., ethyl acetate; nitriles, e.g., acetonitrile or isobutyronitrile; amide, e.g., dimethylformamide or dimethylacetamide; plant oil, e.g., soybean oil or cottonseed oil; dimethyl sulfoxide; or water.

Gaseous carriers include, for example, LPG, air, nitrogen, carbon dioxide, and dimethyl ether.

Surfactants or dispersants usable for emulsifying, dispersing, or spreading include, for example, alkylsulfuric esters, alkyl (aryl) sulfonates, polyoxyalkylene alkyl (aryl) ethers, polyol esters, and lignosulfonates. Adjuvants usable for improving the properties of formulations include, for example, carboxymethylcellulose, gum arabic, polyethylene glycol, and calcium stearate.

The above carriers, surfactants, dispersants, and adjuvants may be used either alone or in combination as needed.

The content of the active ingredient in these formulations is not particularly limited but is preferably 1-75% by weight for emulsifiable concentrate, 0.3-25% by weight for dustable powder, 1-90% by weight for wettable powder, and 0.5-10% by weight for granules.

According to another aspect of the present invention, there is provided a method for controlling a harmful organism, comprising applying an effective amount of a compound represented by Formula I or a salt thereof to an object selected from water surface, soil, nutrient solution in nutriculture, solid medium in nutriculture, and seed, root, tuber, bulb, and rhizome of a plant.

According to one embodiment of the present invention, there is provided a method for controlling a harmful organism, comprising applying an effective amount of a compound represented by Formula I or a salt thereof to the harmful organism or its habitat. According to a preferred embodiment of the present invention, there is provided a method for controlling a harmful organism, comprising applying an effective amount of a compound represented by Formula I or a salt thereof to a plant or soil.

Compounds represented by Formula I or salts thereof directly exert potent control effect against harmful organisms. Further, use of the compounds as a mixture with other harmful organism control agents can be expected to exert higher control effect than the control effect attained when the compounds or other harmful organism control agents are used alone. Thus, according to the present invention, there is provided a harmful organism control composition comprising at least one of compounds represented by Formula I or salts thereof and at least one other harmful organism control agent. Further, according to another embodiment of the present invention, there is provided use of the harmful organism control composition for the protection of useful plants (cultivated plants) from harmful organisms. Furthermore, according to another embodiment, there is provided use of the harmful organism control composition in the manufacture of the agent used for the protection of useful plants (cultivated plants) from harmful organisms.

Compositions or compounds or admixtures thereof with other harmful organism control agents usable as harmful organism control agents according to the present invention are used for the control of many insect pests for a variety of plants. Target plants include wheat and barley, coarse cereals such as corn, millet, common millet, barnyard millet and edible sorghum, fruit trees such as oranges, apples and grapes, vegetables such as cucumbers, pumpkins, melons, cabbages, eggplants, tomatoes and strawberries, tubers such as potatoes, sweet potatoes and taros, pulses such as

azuki beans, kidney bean and soybeans, oil crops such as rapeseeds, feed crops such as grazing, sorghum and corn used for animal feed, ornamental plants, foliage plants, timbers, tea, sugar beet, sugarcanes, sunflower, hops, cotton plants, nicotiana, Arabian coffee, lawn grass, and champignon.

Compositions or compounds or admixtures thereof with other harmful organism control agents usable as harmful organism control agents of the present invention can be applied to insect pests, plants, and plant propagation materials, specifically, for example, seeds, plant foliage, roots, soil, water surface, culture materials, and room where the entry of insect pests should be prevented. The treatment by the compounds, admixtures, and composition of the present invention may be carried out before and after the entry of insect pests.

The present invention encompasses disinfestation of harmful animal-parasitic organisms. The disinfestation of harmful organisms can be carried out by application to habitats where harmful animal-parasitic organisms grow or would grow, animal farming places, feed, plants, seeds, soil, materials and growth environments, or materials, plants, seeds, soil, and water surface where the entry of harmful animal-parasitic organisms should be prevented.

Plant propagation materials as objects to which the present invention is applied refer to plants having an ability of reproduction used in plant growth, including, but are not limited to, seeds, slash or lops, a pullout portion of a part of a tuber, specifically seeds, roots, fruits, tubers, bulbs, corms, roots, shoots, and sprouts. Seedlings or juvenile plants that have been transplanted after budding or rooting are also included. A plant protecting agent is applied for prevention purposes to these plant propagation materials at the time of settled plating or transplanting.

The term "cultivated plants" is to be understood as including plants which have been modified by breeding, mutagenesis and/or genetic engineering. Genetically modified plants (GMO) are plants, of which genetic material has been so modified by the use of recombinant DNA techniques that under natural circumstances cannot readily be obtained by cross breeding, mutations or natural recombination. Normally one or more genes are integrated into the genetic material of a genetically modified plant to improve certain performances of the plant. Such genetic modifications also include but are not limited to targeted post-translational modification of protein(s) (oligo- or polypeptides), for example by glycosylation or polymer additions such as prenylated, acetylated or farnesylated moieties or PEG moieties (e.g., as disclosed in Biotechnol Prog. 2001 July-August; 17(4):720-8, Protein Eng Des Sel. 2004 January; 17(1):57-66, Nat. Protoc. 2007; 2(5):1225-35, Curr Opin Chem. Biol. 2006 October; 10(5):487-91. Epub 2006 Aug. 28, Biomaterials. 2001 March; 22(5):405-17, Bioconjug Chem. 2005 Jan.-Feb.; 16(1):113-21).

The term "cultivated plants" is to be understood as also including plants that have been rendered tolerant to applications of specific classes of herbicides, such as hydroxyphenyl pyruvate dioxygenase (HPPD) inhibitors; acetolactate synthase (ALS) inhibitors such as sulfonylureas (see e.g., US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073) or imidazolinones (see, e.g., US 6,222,100, WO 01/82685, WO 00/26390, WO97/41218, WO 98/02526, WO 98/02527, WO 04/106529, WO 05/20673, WO 03/14357, WO 03/13225, WO 03/14356, WO 04/16073); enolpyruvylshikimate-3-phosphate synthase (EPSPS) inhibitors such as glyphosate (see, e.g., WO 92/00377); glutamine synthase (GS) inhibitors such as glufosinate (see, e.g., EP-A-0242236, EP-A-242246) or oxynil herbicides (see, e.g., US 5,559,024). Plants resistant to these herbicides can be obtained by conventional methods of breeding or genetic engineering.

Several cultivated plants have been rendered tolerant to herbicides by conventional methods of breeding (mutagenesis), for example Clearfield® (registered trademark) summer rape (Canola) being tolerant to imidazolinones, e.g., imazamox. Genetic engineering methods have been used to render cultivated plants, such as soybean, cotton, corn, beets and rape, tolerant to herbicides, such as glyphosate and glufosinate, some of which are commercially available under the trade names Roundup Ready® (registered trademark) (glyphosate-tolerant) and LibertyLink® (registered trademark) (glufosinate-tolerant).

The term "cultivated plants" is to be understood as also including plants that are by the use of recombinant DNA techniques capable of synthesizing one or more insecticidal proteins, such as δ-endotoxins, e.g., CryIA(b), CryIA(c), CryIF, CryIF(a2), CryIIA(b), CryIIIA, CryIIIB(b1) or Cry9c; vegetative insecticidal proteins (VIP), e.g., VIP1, VIP2, VIP3 or VIP3A; insecticidal proteins of bacteria colonizing nematodes, for example *Photorhabdus* spp. or *Xenorhabdus* spp.; toxins produced by animals, such as scorpion toxins, arachnid toxins, wasp toxins, or other insect-specific neurotoxins; toxins produced by fungi, such as Streptomycetes toxins; plant lectins such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases.

In the context of the present invention, these insecticidal proteins or toxins are to be also understood expressly as pre-toxins, hybrid proteins, truncated or otherwise modified proteins.

Hybrid toxins are produced by a recombinant technique using a new combination of protein domains (see, for example WO 02/015701). Further examples of such toxins or genetically modified plants capable of synthesizing such

toxins are disclosed, for example, in EP-A 374 753, WO 93/007278, WO 95/34656, EP-A 427 529, EP-A 451 878, WO 03/018810 and WO 03/052073. The methods for producing such genetically modified plants are generally known to a person skilled in the art and are described, for example, in the publications mentioned above.

These insecticidal proteins contained in the genetically modified plants impart to the plants producing these proteins tolerance against insect pests from certain taxonomic groups of arthropods, particularly to beetles (Coleoptera), flies (Diptera), and butterflies and moths (Lepidoptera) and to plant-parasitic nematodes (Nematoda).

The term "cultivated plants" is to be understood as also including plants that are by the use of recombinant DNA techniques capable of synthesizing one or more proteins to increase the tolerance of those plants to bacterial, viral or fungal pathogens. Examples of such proteins are the so-called "pathogenesis-related proteins" (PR proteins, see, for example EP-A 0 392 22), plant disease resistance genes (for example potato cultivars, which express resistance genes acting against *Phytophthora infestans* derived from the Mexican wild potato *Solanum bulbocastanum)* or T4-lysozyme (e.g., potato cultivars capable of synthesizing the proteins with increased resistance against bacteria such as *Erwinia amylvora).* The methods for producing such genetically modified plants are generally known to a person skilled in the art and are described, for example, in the publications mentioned above.

The term "cultivated plants" is to be understood as also including plants that are by the use of recombinant DNA techniques capable of synthesizing one or more proteins to increase the productivity (e.g., biomass production, grain yield, sugar content, oil content or protein content), tolerance to drought, salinity or other growth-limiting environmental factors, or tolerance to pests and fungal, bacterial or viral pathogens.

The term "cultivated plants" is to be understood as also including plants that contain by the use of recombinant DNA techniques a modified amount of substances of content or new substances of content, specifically to improve human or animal nutrition, for example oil crops that produce health-promoting long-chain omega-3 fatty acids or unsaturated omega-9 fatty acids (e.g., Nexera® (registered trademark) rape).

The term "cultivated plants" is to be understood as also including plants that contain by the use of recombinant DNA techniques a modified amount of substances of content or new substances of content, specifically to improve raw material production, for example, potatoes that produce increased amounts of amylopectin (e.g., Amflora® (registered trademark) potato).

Preferred methods for applying the compounds of Formula I or compositions comprising the compounds for use as harmful organism control agents to plants or soil include spreading treatment, soil treatment, surface treatment, or fumigation treatment. Examples of spreading treatment include spreading, spraying, misting, atomizing, granule application, or water surface application. Examples of soil treatment include soil drenching or soil mixing. Examples of surface treatment include coating, dust coating, or covering. Further, examples of fumigation treatment include covering of soil with polyethylene film after soil injection. Accordingly, the control method of the present invention also includes a method in which a compound represented by Formula I or a preparation comprising the compound is applied by fumigation treatment in a closed space.

Other harmful organism control agents admixable into compounds represented by Formula I or salts thereof include insecticides, bactericides, miticides or tickicides, herbicides, and plant growth-regulating agents. Specific agents include those described, for example, in The Pesticide Manual, 13th edition, published by The British Crop Protection Council; and SHIBUYA INDEX, the 14th edition, 2009, published by SHIBUYA INDEX RESEARCH GROUP. More specific examples thereof are M.1.-M.27. described below:

M.1. Organophosphate insecticides: acephate, azamethiphos, azinphos-ethyl, azinphos-methyl, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, flupyrazophos, fosthiazate, heptenophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;

M.2. Carbamate insecticides: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, and triazamate;

M.3. Synthetic pyrethroid insecticides: acrinathrin, allethrin, *d-cis-trans*-allethrin, d-trans-allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, imiprothrin, metofluthrin, permethrin, phenothrin, prallethrin, profluthrin, pyrethrins (pyrethrum), resmethrin, silafluofen, tefluthrin, tetramethrin, tralomethrin, and transfluthrin;

M.4. Juvenile hormone mimics: hydroprene, kinoprene, methoprene, fenoxycarb, and pyriproxyfen;

M.5. Nicotinic receptor agonist/antagonist compounds: acetamiprid, bensultap, cartap hydrochloride, clothianidin, dinotefuran, imidacloprid, thiamethoxam, nitenpyram, nicotine, spinosad (allosteric agonist), spinetoram (allosteric agonist), thiacloprid, thiocyclam, thiosultap-sodium, and AKD 1022;

M.6. GABA-gated chloride channel antagonist compounds: chlordane, endosulfan, gamma-HCH, ethiprole, fipronil, pyrafluprole, and pyriprole;

M.7. Chloride channel activators: abamectin, emamectin benzoate, milbemectin, lepimectin;

M.8. METI I compounds: fenazaquin, fenpyroximate, pyrimidifen, pyridaben, tebufenpyrad, tolfenpyrad, flufenerim, rotenone;

M.9. METI II compounds: acequinocyl, fluacyprim, and hydramethylnon;

M.10. Uncouplers of oxidative phosphorylation: chlorfenapyr, DNOC;

M.11. Other inhibitors of oxidative phosphorylation: azocyclotin, cyhexatin, diafenthiuron, fenbutatin oxide, propargite, and tetradifon;

M.12. Moulting disruptors: cryomazine, chromafenozide, halofenozide, methoxyfenozide, and tebufenozide;

M.13. Synergists: piperonyl butoxide, tribufos;

M.14. Sodium channel blocker compounds: indoxacarb and metaflumizone;

M.15. Fumigants: methyl bromide, chloropicrin, sulfuryl fluoride;

M.16. Selective feeding blockers: cryolite, pymetrozine, and flonicamid;

M.17. Mite growth inhibitors: clofentezine, hexythiazox, and etoxazole;

M.18. Chitin synthesis inhibitors: buprofezin, bistrifluron, chlorfluazuron, diflubenzuron, flucycloxuron, flufenoxuron, hexaflumuron, lufenuron, novaluron, noviflumuron, teflubenzuron, and triflumuron;

M.19. Lipid biosynthesis inhibitors: spirodiclofen, spiromesifen, and spirotetramat;

M.20. Octapaminergic agonsits: amitraz;

M.21. Ryanodine receptor modulators: flubendiamide and phtalamid compound (R)-,(S)-3-chloro-N1-{2-methyl-4-[1,2,2,2-tetrafluoro-1-(trifluoromethyl)ethyl]phenyl}-N2-(1-methyl-2-methylsulfonylethyl)phthalamide (M21.1);

M.22. Isoxazoline compounds: 4-[5-(3,5-dichloro-phenyl)-5-trifluoromethyl-4,5-dihydro-isoxazol-3-yl]-2-methyl-N-pyridin-2-ylmethyl-benzamide (M22.1), 4-[5-(3,5-dichloro-phenyl)-5-trifluoromethyl-4,5-dihydro-isoxazol-3-yl]-2-methyl-N-(2,2,2-trifluoroethyl)-benzamide (M22.2), 4-[5-(3,5-dichloro-phenyl)-5-trifluoromethyl-4,5-dihydro-isoxazol-3-yl]-2-methyl-N-[(2,2,2-trifluoroethylcarbamoyl)-methyl]-benzamide (M22.3), 4-[5-(3,5-dichloro-phenyl)-5-trifluoromethyl-4,5-dihydro-isoxazol-3-yl]-naphthalene-1-carboxylic acid [(2,2,2-trifluoroethylcarbamoyl)-methyl]-amide (M22.4), 4-[5-(3,5-dichlorophenyl)-5-trifluoromethyl-4,5-dihydro-isoxazol-3-yl]-N-[(methoxyimino)methyl]-2-methylbenzamide (M22.5), 4-[5-(3-chloro-5-trifluoromethyl-phenyl)-5-trifluoromethyl-4,5-dihydro-isoxazol-3-yl]-2-methyl-N-[(2,2,2-trifluoroethylcarbamoyl)-methyl]-benzamide (M22.6), 4-[5-(3-chloro-5-trifluoromethyl-phenyl)-5-trifluoromethyl-4,5-dihydro-isoxazol-3-yl]-naphthalene-1-carboxylic acid [(2,2,2-trifluoroethylcarbamoyl)-methyl]-amide (M22.7) and 5-[5-(3,5-dichloro-4-fluorophenyl)-5-trifluoromethyl-4,5-dihydro-isoxazol-3-yl]-2-[1,2,4]triazol-1-yl-benzonitrile (M22.8);

M.23. Anthranilamide compounds: chloranthraniliprole, cyantraniliprole; 5-bromo-2-(3-chloro-pyridin-2-yl)-2H-pyrazole-3-carboxylic acid [4-cyano-2-(1-cyclopropyl-ethylcarbamoyl)-6-methylphenyl]-amide (M23.1), 5-bromo-2-(3-chloro-pyridin-2-yl)-2H-pyrazole-3-carboxylic acid [2-chloro-4-cyano-6-(1-cyclopropyl-ethylcarbamoyl)-phenyl]-amide (M23.2), 5-bromo-2-(3-chloro-pyridin-2-yl)-2H-pyrazole-3-carboxylic acid [2-bromo-4-cyano-6-(1-cyclopropyl-ethylcarbamoyl)-phenyl]-amide (M23.3), 5-bromo-2-(3-chloro-pyridin-2-yl)-2H-pyrazole-3-carboxylic acid [2-bromo-4-chloro-6-(1-cyclopropyl-ethylcarbamoyl)-phenyl]-amide (M23.4), 5-bromo-2-(3-chloro-pyridin-2-yl)-2H-pyrazole-3-carboxylic acid [2,4-dichloro-6-(1-cyclopropyl-ethylcarbamoyl)-phenyl]-amide (M23.5), 5-bromo-2-(3-chloro-pyridin-2-yl)-2H-pyrazole-3-carboxylic acid [4-chloro-2-(1-cyclopropyl-ethylcarbamoyl)-6-methylphenyl]-amide (M23.6), N'-(2-{[5-bromo-2-(3-chloro-pyridin-2-yl)-2H-pyrazole-3-carbonyl]-amino}-5-chloro-3-methylbenzoyl)-hydrazinecarboxylic acid methyl ester (M23.7), N'-(2-{[5-bromo-2-(3-chloro-pyridin-2-yl)-2H-pyrazole-3-carbonyl]-amino}-5-chloro-3-methylbenzoyl)-N'-methylhydrazinecarboxylic acid methyl ester (M23.8), N'-(2-{[5-bromo-2-(3-chloro-pyridin-2-yl)-2H-pyrazole-3-carbonyl]-amino}-5-chloro-3-methylbenzoyl)-N,N'-dimethylhydrazinecarboxylic acid methyl ester (M23.9), N'-(3,5-dibromo-2-{[5-bromo-2-(3-chloro-pyridin-2-yl)-2H-pyrazole-3-carbonyl]-amino}-benzoyl)-hydrazinecarboxylic acid methyl ester (M23.10), N'-(3,5-dibromo-2-{[5-bromo-2-(3-chloro-pyridin-2-yl)-2H-pyrazole-3-carbonyl]-amino}-benzoyl)-N'-methylhydrazinecarboxylic acid methyl ester (M23.11) and N'-(3,5-dibromo-2-{[5-bromo-2-(3-chloro-pyridin-2-yl)-2H-pyrazole-3-carbonyl]-amino}-benzoyl)-N,N'-dimethylhydrazinecarboxylic acid methyl ester (M23.12);

M.24. Malononitrile compounds: 2-(2,2,3,3,4,4,5,5-octafluoropentyl)-2-(3,3,3-trifluoropropyl)-malononitrile ($CF_2H-CF_2-CF_2-CF_2-CH_2-C(CN)_2-CH_2-CH_2-CF_3$) (M24.1) and 2-(2,2,3,3,4,4,5,5-octafluoropentyl)-2-(3,3,4,4,4-pentafluorobutyl)-malonodinitrile ($CF_2H-CF_2-CF_2-CF_2-CH_2-C(CN)_2-CH_2-CH_2-CF_2-CF_3$) (M24.2);

M.25. Microbial disruptors: *Bacillus thuringiensis* subsp.*Israelensi, Bacillus sphaericus, Bacillus thuringiensis subsp.Aizawai, Bacillus thuringiensis subsp.Kurstaki, Bacillus thuringiensis* subsp.*Tenebrionis*;

M.26. Aminofuranone compounds: 4-{[(6-bromopyrid-3-yl)methyl](2-fluoroethyl)amino}furan-2(5H)-on (M26.1), 4-{[(6-fluoropyrid-3-yl)methyl](2,2-difluoroethyl)amino}furan-2(5H)-on (M26.2), 4-{[(2-chloro-1,3-thiazolo-5-yl)methyl](2-fluoroethyl)amino}furan-2(5H)-on (M26.3), 4-{[(6-chloropyrid-3-yl)methyl](2-fluoroethyl)amino}furan-2(5H)-on (M26.4), 4-{[(6-chloropyrid-3-yl)methyl](2,2-difluoroethyl)amino}furan-2(5H)-on (M26.5), 4-{[(6-chloro-5-fluoropyrid-3-yl)methyl](methyl)amino}furan-2(5H)-on (M26.6), 4-{[(5,6-dichloropyrid-3-yl)methyl](2-fluoroethyl)amino}furan-2(5H)-on (M26.7), 4-{[(6-chloro-5-fluoropyrid-3-yl)methyl](cyclopropyl)amino}furan-2(5H)-on (M26.8), 4-{[(6-chloropyrid-3-yl)methyl](cyclopropyl)amino}furan-2(5H)-on (M26.9) and 4-{[(6-chloropyrid-3-yl)methyl](methyl)amino}furan-2(5H)-on (M26.10);

M.27. Other insecticides: aluminium phosphide, amidoflumet, benclothiaz, benzoximate, bifenazate, borax, bromopropylate, cyanide, cyenopyrafen, cyflumetofen, chinomethionat, dicofol, fluoroacetate, phosphine, pyridalyl, pyrifluquinazon, sulfur, organic sulfur compounds, tartar emetic, sulfoxaflor, N-R'-2,2-dihalo-1-R'' cyclopropanecarboxamide-2-(2,6-dichloro-$\alpha,\alpha,\alpha$-trifluoro-*p*-tolyl)hydrazone or N-R'-2,2-di(R''')propionamide-2-(2,6-dichloro-$\alpha,\alpha,\alpha$-trifluoro-*p*-tolyl)-hydrazone, wherein R' is methyl, ethyl, or a halogen selected from chloro and bromo; R'' is hydrogen atom or methyl and R''' is methyl or ethyl, 4-but-2-ynyloxy-6-(3,5-dimethyl-piperidin-1-yl)-2-fluoropyrimidine (M27.1), cyclopropaneacetic acid, 1,1'-[(3S,4R,4a R,6S,6aS,12R,12aS,12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl]ester (M27.2) and 8-(2-cyclopropylmethoxy-4-trifluoromethylphenoxy)-3-(6-trifluoromethylpyridazin-3-yl)-3-azabicyclo[3.2.1]octane (M27.3).

Paraoxon and preparation thereof has been described in Farm Chemicals Handbook, Volume 88, Meister Publishing Company, 2001. Flupyrazophos has been described in Pesticide Science, 54, 1988, p. 237-243 and US 4822779. AKD 1022 and preparation thereof has been described in US 6300348. The anthranilamides M23.1-M23.6 have been described in WO 2008/72743 and WO 200872783, and those M23.7-M23.12 have been described in WO2007/043677. The phthalamide M21.1 is known from WO 2007/101540. The alkynylether compound M27.1 is described e.g. in JP 2006131529. Organic sulfur compounds have been described in WO 2007060839. The isoxazoline compounds M22.1-M22.8 have been described in e.g. WO 2005/085216, WO 2007/079162, WO 2007/026965, WO 2009/126668 and WO2009/051956. The aminofuranone compounds M26.1-M26.10 have been described, for example, in WO 2007/115644. The pyripyropene derivative M27.2 has been described in WO 2008/66153 and WO 2008/108491. The pyridazin compound M27.3 has been described in JP 2008/115155. Malononitrile compounds such as those (M24.1) and (M24.2) have been described in WO 02/089579, WO 02/090320, WO 02/090321, WO 04/006677, WO 05/068423, WO 05/068432, and WO 05/063694.

In one specific embodiment, the insecticidal composition comprises a biologically effective amount of at least one

of the pyripyropene derivatives (Component A) and at least one additional active ingredient (Component B) selected from the following compounds:

(1) Acetylcholinesterase (AChE) inhibitors: chlorpyrifos (CAS No.: 2921-88-2), acephate (CAS No.: 30560-19-1);

(2) γ-Aminobutyric acid (GABA)-gated chloride channel antagonist: fipronil (CAS No.: 120068-37-3);

(3) Sodium channel modulators: lambda-cyhalothrin (CAS No.: 91465-08-6), bifenthrin (CAS No.: 82657-04-3), deltamethrin (CAS No.: 52918-63-5), beta-cypermethrin (CAS No.: 1224510-29-5), alpha-cypermethrin (CAS No.: 67375-30-8);

(4) Nicotinic acetylcholine receptor (nAChR) competitive modulators: imidacloprid (CAS No.: 138261-41-3), thiamethoxam (CAS No.: 153719-23-4), acetamiprid (CAS No.: 135410-20-7), dinotefuran (CAS No.: 165252-70-0), clothianidin (CAS No.: 210880-92-5), sulfoxaflor (CAS No.: 946578-00-3), flupyradifurone (CAS No.: 951659-40-8), thiacloprid (CAS No.: 111988-49-9), triflumezopyrim (CAS No.: 1263133-33-0), paichongding (CAS No.: 948994-16-9);

(5) Glutamate-gated chloride channel (GluCl) allosteric modulators: abamectin (CAS No.: 71751-41-2), emamectin benzoate (CAS No.: 155569-91-8);

(6) Juvenile hormone mimics: pyriproxyfen (CAS No.: 95737-68-1);

(7) Nicotinic acetylcholine receptor (nAChR) channel blockers: cartap (CAS No.: 15263-53-3);

(8) Inhibitors of chitin biosynthesis, type 1: buprofezin (CAS No.: 69327-76-0), spiromesifen (CAS No.: 283594-90-1);

(9) Inhibitors of acetyl CoA carboxylase: spirotetramat (CAS No.: 203313-25-1), spiropidion (CAS No.: 1229023-00-0);

(10) Ryanodine receptor modulators: cyantraniliprole (CAS No.: 736994-63-1);

(11) Chordotonal organ modulators - undefined target site: flonicamid (CAS No.: 158062-67-0), dimpropyridaz (CAS No.: 1403615-77-9);

(12) γ-Aminobutyric acid (GABA)-gated chloride channel allosteric modulators: broflanilide (CAS No.: 1207727-04-5), fluxametamide (CAS No.: 928783-29-3), isocycloseram (CAS No.: 2061933-85-3), compound W (CAS No.: 2892524-05-7);

(13) Nicotinic acetylcholine receptor (nAChR) allosteric modulators: spinosad (CAS No.: 168316-95-8);

(14) Others: *Metarhizium anisopliae.*

In another specific embodiment, the weight ratio of active ingredients A to B in the insecticidal composition is 1:150-150:1, 1:100-100:1, 1:80-80:1, 1:60-60:1, 1:40-40:1, 1:20-20:1, 1:10-10:1, 1:8-8:1, 1:5-5:1, or 1:3-1:1.

The term "biologically effective dose" refers to an amount of a biologically active compound (e.g., compound of Formula I) sufficient to produce the desired biological effect when applied to (i.e., contacted with) an insect pest to be controlled, or its environment, or a plant, a seed from which the plant has grown, or the location of the plant (e.g., growth medium), thereby protecting the plant from damages caused by the insect pest or achieving other desired effects (e.g., increasing activity of the plant). The compound of the present invention may also be applied prophylactically where insect pests or parasites are expected to appear.

The present invention also provides a preparation method of the pyripyropene derivative, which is prepared by using

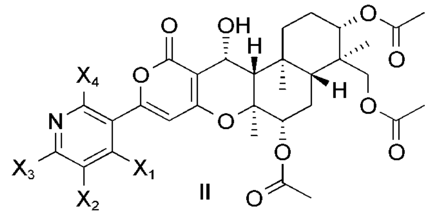

II

as an intermediate.

In one specific embodiment, the method comprises at least one step of the following steps (specific step(s) chosen according to the structure of the target product):

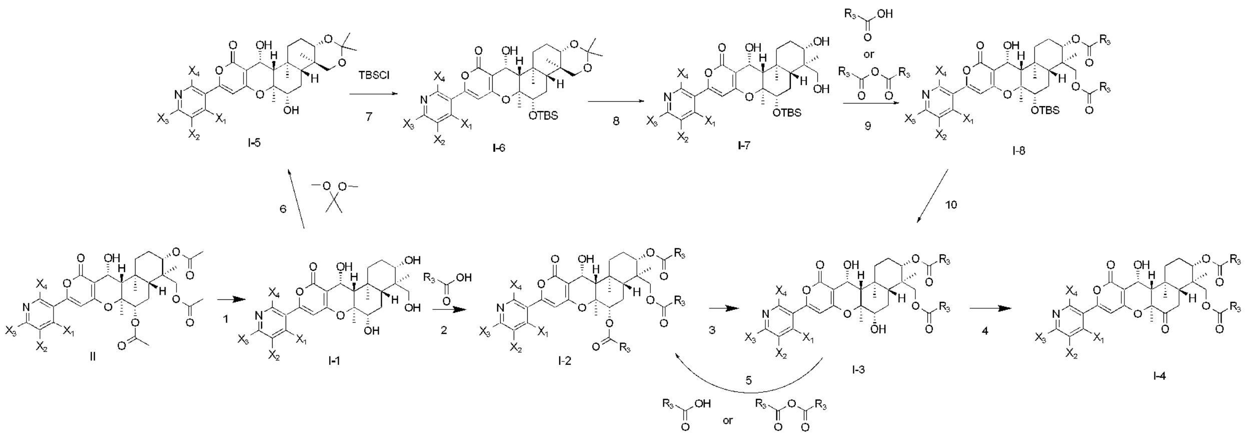


wherein, the definitions of $X_1$, $X_2$, $X_3$, $X_4$ and $R_3$ are as described above.

Wherein, the reactions in steps 1 and 3 are both carried out in the presence of a solvent and a base; the reactions in steps 2, 5, and 9 are carried out in the presence of a solvent; preferably, a condensing agent is added during the reactions in steps 2, 5, and 9, with or without the addition of a base; the reaction in step 4 is carried out in the presence of an oxidizing agent and a solvent; the reaction in step 6 is carried out in the presence of an acid catalyst and a solvent; the reaction in step 7 is carried out in the presence of an organic base and a solvent; the reaction in step 8 is carried out in the presence of an acid (e.g., acetic acid) and a solvent; the reaction in step 10 is carried out in the presence of a pyridine hydrofluoride and a solvent.

In another specific embodiment, the solvent is selected from at least one of water, THF, pyridine, DMF, DMA, methanol, ethanol, acetonitrile, dichloroethane, DMSO, dioxane, dichloromethane (DCM) and ethyl acetate.

In another specific embodiment, the base is selected from at least one of inorganic bases (e.g., $K_2CO_3$, $Na_2CO_3$, $Cs_2CO_3$, $NaHCO_3$, $KHCO_3$, KF, CsF, KI, NaI, $K_3PO_4$, $K_2HPO_4$, NaOH, KOH, NaH, KH, etc.) and organic bases (e.g., DBU, DMAP, imidazole, pyrazole, triethylamine, DIEA, potassium trimethylsilanolate, AcOK, AcONa, MeONa, EtONa, t-BuONa, etc.).

In another specific embodiment, the condensing agent is selected from at least one of Py-BOP, Py-AOP, HOBT (1-hydroxybenzotriazole), EDCI (1-(3-dimethylaminopropyl)-3-ethylcarbodiimide hydrochloride), DMAP (4-dimethylaminopyridine), DCC, HBTU and HATU.

In another specific embodiment, the oxidizing agent is Dess-Martin periodinane or PCC (pyridinium chlorochromate) oxidizing agent.

In another specific embodiment, the acid catalyst is pyridinium p-toluenesulfonate (PPTS) or p-toluenesulfonic acid.

In another specific embodiment, the compound II is prepared by fermentation culture using *Neosartorya pseudofischeri* with 5-fluoronicotinic acid as a substrate.

In addition, the compounds of the present invention can be prepared with reference to the methods set forth in WO2011093186, WO2010010955, WO2011148886, etc.

The present invention also provides an intermediate as shown in Formula II.

The present invention also provides a preparation method of the pyripyropene derivative, which is prepared through reduction reaction of

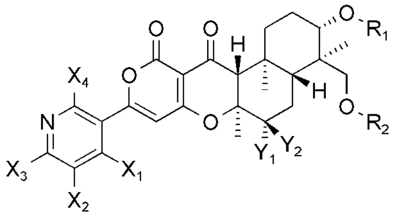

III ,

wherein, the definitions of $X_1$, $X_2$, $X_3$, $X_4$, $Y_1$, $Y_2$, $R_1$, and $R_2$ are as describe above.

In one specific embodiment, the reduction reaction is carried out in the presence of sodium borohydride and a solvent, preferably, cerium trichloride may be added.

In another specific embodiment, the solvent is selected from at least one of THF, DMF, DMA, methanol, ethanol, acetonitrile, dichloroethane, DMSO, dioxane, dichloromethane, and ethyl acetate.

The present invention also provides an intermediate as shown in Formula III.

According to another aspect of the present invention, there is provided use of a compound represented by Formula I or an agriculturally or animal husbandry-acceptable salt thereof as a harmful organism control agent.

Detailed Embodiments of the Invention

The following examples are used to illustrate the present invention and should not be construed as limiting the present invention in any way. The scope of rights claimed by the present invention is described in the claims.

In view of the economy and diversity of the compounds, some compounds were preferably synthesized. Among the many synthesized compounds, selected ones are listed in Table 1 below. The specific compound structure and corresponding compound information are as set forth in Table 1. The compounds in Table 1 are only to better illustrate the present invention but do not limit the present invention. For a person skilled in the art, it should not be understood that the scope of the above-mentioned subject matters of the present invention is limited to the following compounds.

Table 1 Compound structure and $^1$H NMR thereof

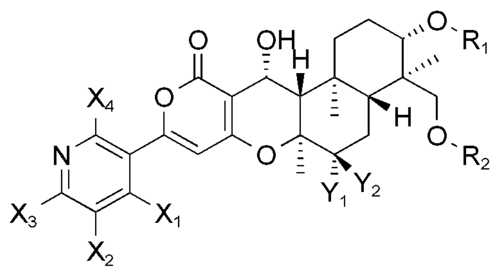

I

| No. | $X_1$ | $X_2$ | $X_3$ | $X_4$ | $Y_1$ | $Y_2$ | $R_1$ | $R_2$ | $^1$H NMR |
|---|---|---|---|---|---|---|---|---|---|
| 1 | F | H | H | H | OH | H | $COCH_3$ | $COCH_3$ | |
| 2 | F | H | H | H | OH | H | $COCH_2CH_3$ | $COCH_2CH_3$ | |
| 3 | F | H | H | H | OH | H | 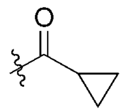 | 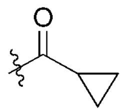 | |
| 4 | H | F | H | H | OH | H | $COCH_3$ | $COCH_3$ | |
| 5 | H | F | H | H | OH | H | 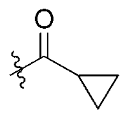 | 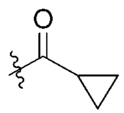 | $^1$H NMR ($CDCl_3$-*d*, 300 MHz) $\delta_H$ 8.81 (1H, br s), 8.54 (1H, br s), 7.83 (1H, br d), 6.54 (1H, s), 4.99-4.98 (1H, m), 4.81 (1H, dd, *J* = 11.1, 5.3 Hz), 3.89 (1H, d, *J* = 11.7Hz), 3.80-3.77 (1H, m), 3.73 (1H, d, *J* = 11.7Hz), 2.90 (1H, br s), 2.28 (1H, br s), 2.16-2.13 (1H, m), 1.90-1.80 (3H, m), 1.65 (3H, s), 1.62-1.55 (3H, m), 1.49-1.43 (2H, m), 1.42 (3H, s), 1.35-1.33 (1H, m), 1.00-0.95 (4H, m), 0.91 (3H, s), 0.88-0.83 (4H, m). |
| 6 | H | F | H | H | OH | H | $COCH_2CH_3$ | $COCH_2CH_3$ | $^1$H NMR (300 MHz, Chloroform-d) δ 8.86-8.80 (m, 1H), 8.55 (d, *J* = 2.6 Hz, 1H), 7.88-7.79 (m, 1H), 6.54 (s, 1H), 4.98 (s, 1H), 4.79 (dd, *J* = 11.0, 5.3 Hz, 1H), 3.80-3.76 (m, 2H), 2.87 (s, 1H), 2.41-2.25 (m, 4H), 2.24-2.11 (m, 2H), 1.94-1.76 (m, 3H), 1.65 (s, 3H), 1.41 (s, 3H), 1.13 (t, *J* = 7.5 Hz, 6H), 0.93-0.78 (m, 8H). |
| 7 | H | H | F | H | OH | H | $COCH_3$ | $COCH_3$ | |
| 8 | H | H | F | H | OH | H | $COCH_2CH_3$ | $COCH_2CH_3$ | |

(continued)

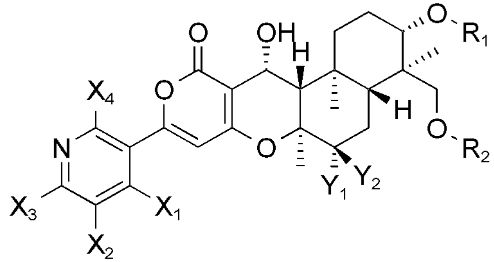

I

| No. | $X_1$ | $X_2$ | $X_3$ | $X_4$ | $Y_1$ | $Y_2$ | $R_1$ | $R_2$ | $^1$H NMR |
|---|---|---|---|---|---|---|---|---|---|
| 9 | H | H | F | H | OH | H | 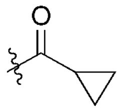 | 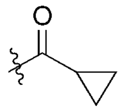 | $^1$H NMR ($CDCl_3$-*d*, 300 MHz) $\delta_H$ 8.63 (1H, d), 8.18 (1H, m), 7.04 (1H, dd, J = 8.7, 2.9 Hz,), 6.45 (1H, s), 4.97 (1H, d), 4.80 (1H, dd, J = 11.1, 5.3 Hz), 3.86 (1H, d, J = 11.7 Hz,), 3.81-3.75 (1H, m), 3.72 (1H, d, J = 11.7 Hz), 2.89 (1H, br s), 2.28 (1H, br s), 2.18-2.09 (1H, m), 1.90-1.77 (3H, m), 1.64 (3H, s), 1.61-1.52 (3H, m), 1.45-1.42 (2H, m), 1.41 (3H, s), 1.35-1.31 (1H, m), 1.01-0.93 (4H, m), 0.90 (3H, s), 0.88-0.83 (4H, m). |
| 10 | H | H | H | F | OH | H | $COCH_3$ | $COCH_3$ | |
| 11 | H | H | H | F | OH | H | $COCH_2CH_3$ | $COCH_2CH_3$ | |
| 12 | H | H | H | F | OH | H | 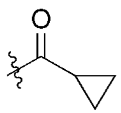 | 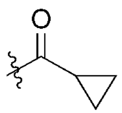 | $^1$H NMR (300 MHz, Chloroform-d) $\delta_H$ 8.41 (1H, m), 8.27 (1H, d), 7.39-7.33 (1H, m), 6.81 (1H, s), 4.98 (1H, d), 4.80 (1H, dd, *J* = 11.1, 5.3 Hz), 3.79 (3H, m), 2.84 (1H, br s), 2.14 (1H, m), 2.04 (1H, s), 1.74-1.90 (3H, m), 1.65 (3H, s), 1.62-1.54 (3H, m), 1.46-1.43 (2H, m), 1.41 (3H, s), 1.37-1.32 (1H, m), 1.02-0.97 (4H, m), 0.90 (3H, s), 0.88-0.82 (4H, m). |
| 13 | F | F | H | H | OH | H | $COCH_2CH_3$ | $COCH_2CH_3$ | |
| 14 | F | H | F | H | OH | H | $COCH_2CH_3$ | $COCH_2CH_3$ | |
| 15 | F | H | H | F | OH | H | $COCH_2CH_3$ | $COCH_2CH_3$ | |
| 16 | H | F | F | H | OH | H | $COCH_2CH_3$ | $COCH_2CH_3$ | |
| 17 | H | F | H | F | OH | H | $COCH_2CH_3$ | $COCH_2CH_3$ | |
| 18 | H | H | F | F | OH | H | $COCH_2CH_3$ | $COCH_2CH_3$ | |

(continued)

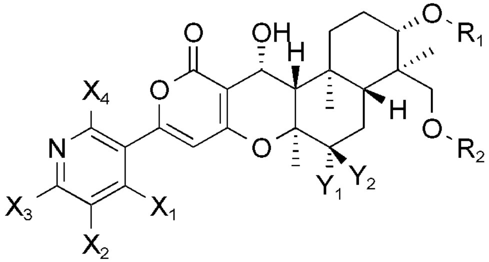

I

| No. | $X_1$ | $X_2$ | $X_3$ | $X_4$ | $Y_1$ | $Y_2$ | $R_1$ | $R_2$ | $^1$H NMR |
|---|---|---|---|---|---|---|---|---|---|
| 19 | F | F | H | H | OH | H | 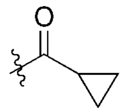 | 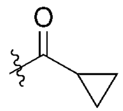 | |
| 20 | F | H | F | H | OH | H | 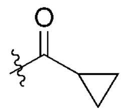 | 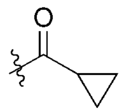 | |
| 21 | F | H | H | F | OH | H | 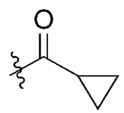 | 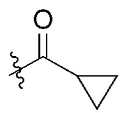 | $^1$H NMR (300 MHz, Chloroform-d) $\delta$ 8.33-8.26 (m, 1H), 7.11 (dd, $J$ = 8.4, 5.7 Hz, 1H), 6.36 (s, 1H), 4.98 (d, $J$ = 4.2 Hz, 1H), 4.81 (dd, $J$ = 11.2, 5.4 Hz, 1H), 3.89-3.70 (m, 3H), 2.50-2.24 (m, 2H), 2.20-2.09 (m, 2H), 1.92-1.79 (m, 2H), 1.67 (s, 3H), 1.63-1.54 (m, 4H), 1.51-1.44 (m, 2H), 1.42 (s, 3H), 1.02-0.94 (m, 4H), 0.91 (s, 3H), 0.88-0.84 (m, 4H). |
| 22 | H | F | F | H | OH | H | 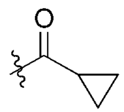 | 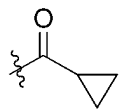 | $^1$H NMR ($CDCl_3$-$d$, 300 MHz) $\delta_H$ 8.39 (1H, t, $J$ = 1.6 Hz), 7.98 (1H, td, $J$ = 9.1, 2.1 Hz), 6.47 (1H, s), 5.00-4.95 (1H, m), 4.81 (1H, dd, $J$ = 11.2, 5.2 Hz), 3.89-3.69 (3H, m), 2.83 (1H, br s), 2.25-2.08 (2H, m), 1.92-1.79 (3H, m), 1.65 (3H, s), 1.63-1.54 (4H, m), 1.46-1.43 (1H, m), 1.42 (3H, s), 1.36-1.32 (1H, m), 1.02-0.94 (4H, m), 0.91 (3H, s), 0.89-0.82 (4H, m). |

(continued)

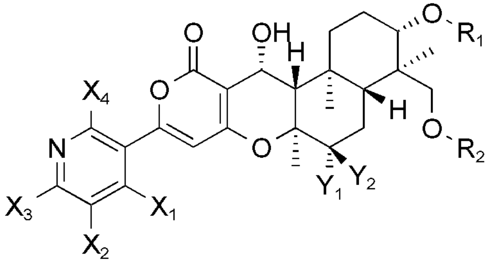

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 23 | H | F | H | F | OH | H | 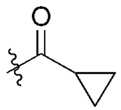 | 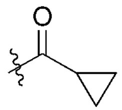 | $^1H$ NMR (300 MHz, Chloroform-d) δ 8.16 (td, $J$ = 7.6, 3.0 Hz, 1H), 8.10 (dd, $J$ = 3.1, 1.7 Hz, 1H), 6.85 (s, 1H), 4.98 (d, $J$ = 4.2 Hz, 1H), 4.81 (dd, $J$ = 11.2, 5.3 Hz, 1H), 3.88-3.72 (m, 3H), 2.34-2.27 (m, 1H), 2.14 (d, $J$ = 13.2 Hz, 2H), 1.92-1.79 (m, 3H), 1.65 (s, 3H), 1.64-1.52 (m, 4H), 1.45 (s, 2H), 1.41 (s, 3H), 1.02-0.93 (m, 4H), 0.91 (s, 3H), 0.85 (m, 4H). |
| 24 | H | H | F | F | OH | H | 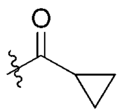 | 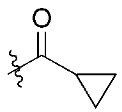 | $^1H$ NMR ($CDCl_3$-$d$, 300 MHz) $\delta_H$ 8.52 (1H, dd, $J$ = 15.9, 8.5 Hz), 7.00 (1H, dd, $J$ = 8.5, 2.8 Hz), 6.73 (1H, s), 4.98 (1H, d, $J$ = 3.8 Hz), 4.81 (1H, dd, $J$ = 11.0, 5.2 Hz), 3.88-3.70 (3H, m), 2.81 (1H, br s), 2.25-2.09 (2H, m), 1.92-1.83 (2H, m), 1.80 1H, s), 1.65 (3H, s), 1.61-1.55 (3H, m), 1.47-1.43 (2H, m), 1.41 (3H, s), 1.35 (1H, s), 1.03-0.93 (4H, m), 0.91 (3H, s), 0.89-0.82 (4H, m). |
| 25 | F | H | H | H | OH | H | 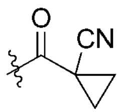 | 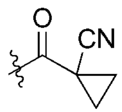 | |
| 26 | H | F | H | H | OH | H | 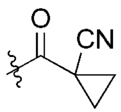 | 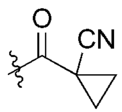 | $^1H$ NMR (300 MHz, Chloroform-d) δ 8.84-8.78 (m, 1H), 8.54 (d, $J$ = 2.7 Hz, 1H), 7.88-7.76 (m, 1H), 6.52 (s, 1H), 4.99-4.90 (m, 1H), 4.84-4.72 (m, 1H), 4.23 (d, $J$ = 11.6 Hz, 1H), 3.99-3.88 (m, 1H), 3.73 (d, $J$ = 11.8 Hz, 1H), 2.88 (s, 1H), 2.30-2.11 (m, 2H), 2.05-1.80 (m, 4H), 1.74-1.59 (m, 12H), 1.49 (s, 1H), 1.45 (s, 3H), 1.41-1.38 (m, 1H), 1.03 (s, 3H). |

(continued)

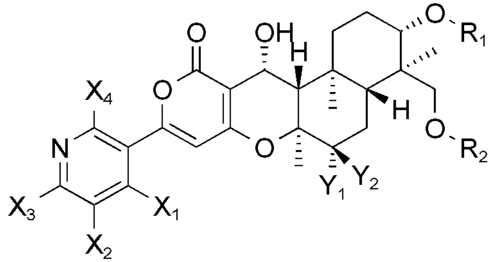

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 27 | H | H | F | H | OH | H | 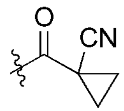 | 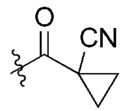 | |
| 28 | H | H | H | F | OH | H | 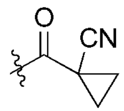 | 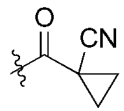 | |
| 29 | F | F | H | H | OH | H | 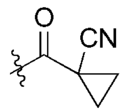 | 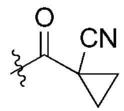 | |
| 30 | F | H | F | H | OH | H | 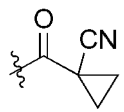 | 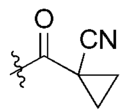 | |
| 31 | F | H | H | F | OH | H | 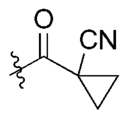 | 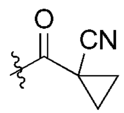 | |
| 32 | H | F | F | H | OH | H | 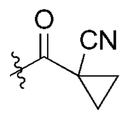 | 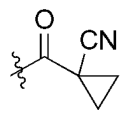 | |

(continued)

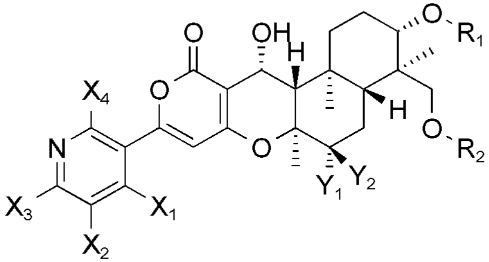

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 33 | H | F | H | F | OH | H | 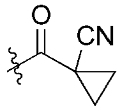 | 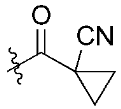 | |
| 34 | H | H | F | F | OH | H | 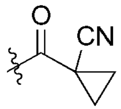 | 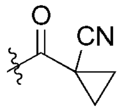 | |
| 35 | F | H | H | H | 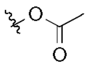 | H | $COCH_3$ | $COCH_3$ | |
| 36 | F | H | H | H | 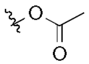 | H | $COCH_2CH_3$ | $COCH_2CH_3$ | |
| 37 | F | H | H | H | 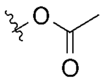 | H | 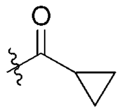 | 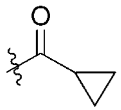 | |
| 38 | H | F | H | H | 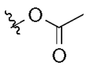 | H | $COCH_3$ | $COCH_3$ | $^1H$ NMR ($CDCl_3$-d, 300 MHz) δH8.83 (1H, brs), 8.55 (1H, brs), 7.82 (1H, brd), 6.48 (1H, s), 5.03-4.98 (2H, m), 4.79 (1H, dd, $J$ = 11.0, 4.9 Hz), 3.79 (1H, d, $J$ = 11.9 Hz), 3.71 (1H, d, $J$ = 11.9 Hz), 2.88 (1H, brs), 2.19-2.14 (4H, m), 2.09 (3H, s), 2.04 (3H, s), 1.92-1.88 (1H, m), 1.85-1.82 (1H, m), 1.80-1.77 (1H, m), 1.69 (3H, s), 1.63-1.60 (2H, m), 1.54-1.53 (1H, m), 1.44 (3H, s), 1.39-1.32 (1H, m), 0.89 (3H, s). |

(continued)

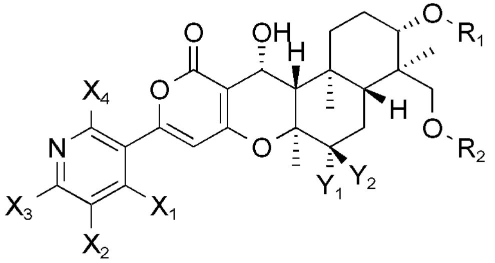

I

| No. | $X_1$ | $X_2$ | $X_3$ | $X_4$ | $Y_1$ | $Y_2$ | $R_1$ | $R_2$ | $^1$H NMR |
|---|---|---|---|---|---|---|---|---|---|
| 39 | H | F | H | H | 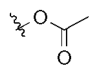 | H | $COCH_2CH_3$ | $COCH_2CH_3$ | |
| 40 | H | F | H | H | 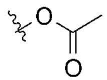 | H | 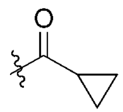 | 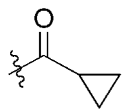 | $^1$H NMR (300 MHz, Chloroform-d) δ 8.85-8.80 (m, 1H), 8.57-8.52 (m, 1H), 7.87-7.79 (m, 1H), 6.48 (s, 1H), 5.07-4.95 (m, 2H), 4.86-4.77 (m, 1H), 3.77 (s, 2H), 2.16 (s, 3H), 1.95-1.78 (m, 4H), 1.69 (s, 3H), 1.65-1.50 (m, 6H), 1.43 (s, 3H), 1.07-0.96 (m, 4H), 0.90 (s, 3H), 0.89-0.80 (m, 4H). |
| 41 | H | H | F | H | 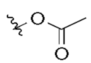 | H | $COCH_3$ | $COCH_3$ | $^1$H NMR (300 MHz, Chloroform-d) δ 8.63 (s, 1H), 8.18 (t, *J* = 9.1 Hz, 1H), 7.08-6.98 (m, 1H), 6.38 (s, 1H), 5.05-4.94 (m, 2H), 4.85-4.72 (m, 1H), 3.84-3.65 (m, 2H), 2.86 (s, 1H), 2.15 (s, 3H), 2.08 (s, 3H), 2.03 (s, 3H), 1.95-1.73 (m, 4H), 1.68 (s, 3H), 1.64-1.51 (m, 3H), 1.43 (s, 3H), 1.33-1.21 (m, 1H), 0.88 (s, 3H). |
| 42 | H | H | F | H | 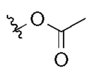 | H | $COCH_2CH_3$ | $COCH_2CH_3$ | |
| 43 | H | H | F | H | 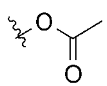 | H | 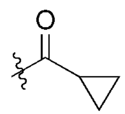 | 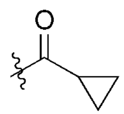 | $^1$H NMR (300 MHz, Chloroform-d) δ 8.63 (s, 1H), 8.18 (ddd, *J* = 8.6, 7.3, 2.5 Hz, 1H), 7.04 (dd, *J* = 8.5, 3.0 Hz, 1H), 6.39 (s, 1H), 5.05-4.94 (m, 2H), 4.80 (dd, *J* = 11.4, 4.9 Hz, 1H), 3.76 (s, 2H), 2.89 (d, *J* = 2.3 Hz, 1H), 2.23-2.10 (m, 4H), 2.04-1.96 (m, 1H), 1.92-1.77 (m, 3H), 1.68 (s, 3H), 1.59-1.49 (m, 5H), 1.43 (s, 3H), 1.07-0.94 (m, 4H), 0.92-0.81 (m, 7H). |
| 44 | H | H | H | F | 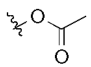 | H | $COCH_3$ | $COCH_3$ | |

(continued)

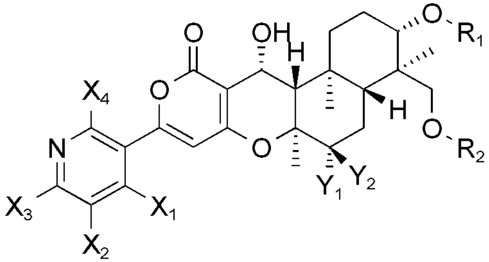

I

| No. | $X_1$ | $X_2$ | $X_3$ | $X_4$ | $Y_1$ | $Y_2$ | $R_1$ | $R_2$ | $^1H$ NMR |
| --- | --- | --- | --- | --- | --- | --- | --- | --- | --- |
| 45 | H | H | H | F | 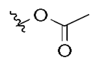 | H | $COCH_2CH_3$ | $COCH_2CH_3$ | |
| 46 | H | H | H | F | 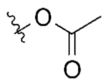 | H | 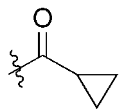 | 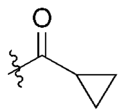 | |
| 47 | F | F | H | H | 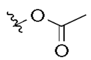 | H | $COCH_2CH_3$ | $COCH_2CH_3$ | |
| 48 | F | H | F | H | 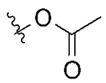 | H | $COCH_2CH_3$ | $COCH_2CH_3$ | |
| 49 | F | H | H | F | 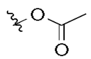 | H | $COCH_2CH_3$ | $COCH_2CH_3$ | |
| 50 | H | F | F | H | 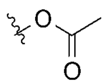 | H | $COCH_2CH_3$ | $COCH_2CH_3$ | |
| 51 | H | F | H | F | 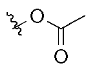 | H | $COCH_2CH_3$ | $COCH_2CH_3$ | |
| 52 | H | H | F | F | 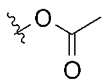 | H | $COCH_2CH_3$ | $COCH_2CH_3$ | |

(continued)

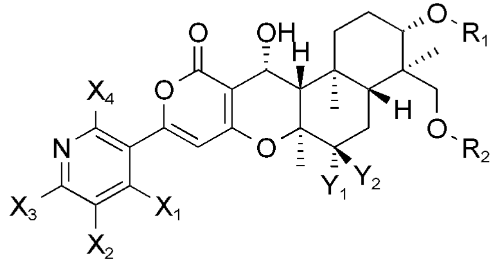

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 53 | F | H | F | H | 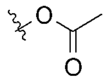 | H | 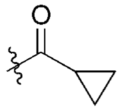 | 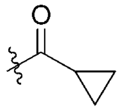 | |
| 54 | F | H | H | F | 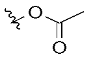 | H | 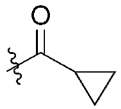 | 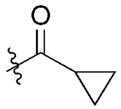 | |
| 55 | H | F | F | H | 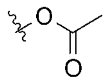 | H | 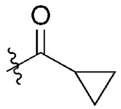 | 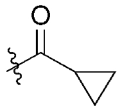 | |
| 56 | H | F | H | F | 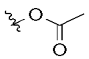 | H | 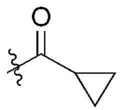 | 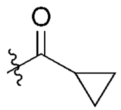 | |
| 57 | H | H | F | F | 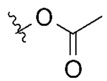 | H | 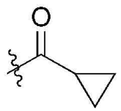 | 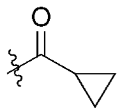 | |
| 58 | F | H | H | H | 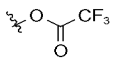 | H | $COCH_2CH_3$ | $COCH_2CH_3$ | |

(continued)

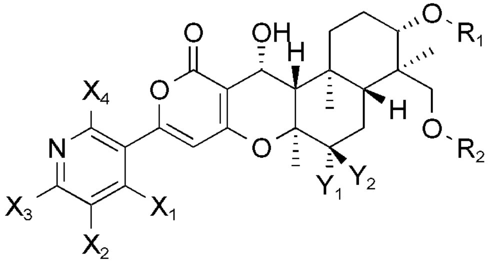

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 59 | F | H | H | H | 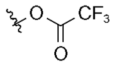 | H | 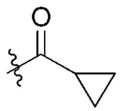 | 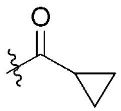 | |
| 60 | H | F | H | H | 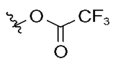 | H | $COCH_2CH_3$ | $COCH_2CH_3$ | $^1H$ NMR (300 MHz, Chloroform-d) δ 8.88-8.80 (m, 1H), 8.58-8.54 (m, 1H), 7.82 (d, *J* = 8.9 Hz, 1H), 6.45 (s, 1H), 5.20-5.06 (m, 1H), 5.00 (d, *J* = 3.6 Hz, 1H), 4.84-4.73 (m, 1H), 3.80 (d, *J* = 12.1 Hz, 1H), 3.71 (d, *J* = 12.2 Hz, 1H), 3.03 (s, 1H), 2.40-2.27 (m, 4H), 2.21-2.12 (m, 1H), 1.92-1.75 (m, 6H), 1.63-1.56 (m, 1H), 1.54 (d, *J* = 4.0 Hz, 1H), 1.45 (s, 3H), 1.42 (s, 1H), 1.39-1.32 (m, 1H), 1.13 (q, *J* = 7.1 Hz, 6H), 0.91 (s, 3H). |
| 61 | H | F | H | H | 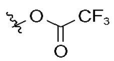 | H | 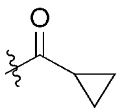 | 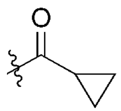 | $^1H$ NMR (300 MHz, Chloroform-d) δ 8.86-8.82 (m, 1H), 8.58-8.53 (m, 1H), 7.86-7.79 (m, 1H), 6.45 (d, *J* = 1.4 Hz, 1H), 5.19-5.11 (m, 1H), 5.03-4.98 (m, 1H), 4.86-4.77 (m, 1H), 3.85-3.74 (m, 2H), 2.88 (s, 1H), 2.18 (d, *J* = 13.4 Hz, 2H), 2.01-1.88 (m, 3H), 1.83 (d, *J* = 12.4 Hz, 2H), 1.73 (s, 3H), 1.66-1.56 (m, 3H), 1.46 (s, 3H), 1.05-0.96 (m, 4H), 0.93 (s, 3H), 0.91-0.82 (m, 4H). |
| 62 | H | H | F | H | 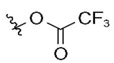 | H | $COCH_2CH_3$ | $COCH_2CH_3$ | |
| 63 | H | H | F | H | 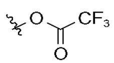 | H | 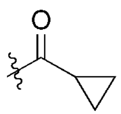 | 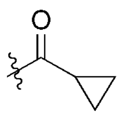 | |
| 64 | H | H | H | F | 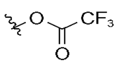 | H | $COCH_2CH_3$ | $COCH_2CH_3$ | |

(continued)

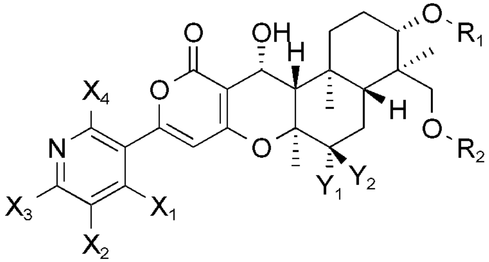

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 65 | H | H | H | F | 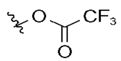 | H | 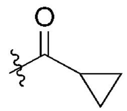 | 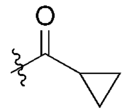 | |
| 66 | F | F | H | H | 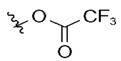 | H | $COCH_2CH_3$ | $COCH_2CH_3$ | |
| 67 | F | H | F | H | 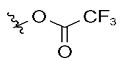 | H | $COCH_2CH_3$ | $COCH_2CH_3$ | |
| 68 | F | H | H | F | 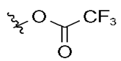 | H | $COCH_2CH_3$ | $COCH_2CH_3$ | |
| 69 | H | F | F | H | 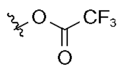 | H | $COCH_2CH_3$ | $COCH_2CH_3$ | |
| 70 | H | F | H | F | 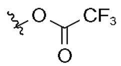 | H | $COCH_2CH_3$ | $COCH_2CH_3$ | |
| 71 | H | H | F | F | 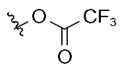 | H | $COCH_2CH_3$ | $COCH_2CH_3$ | |
| 72 | F | F | H | H | 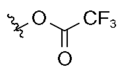 | H | 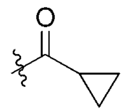 | 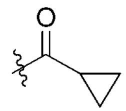 | |

(continued)

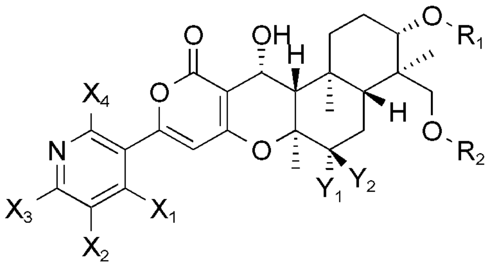

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 73 | F | H | F | H | 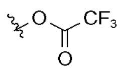 | H | 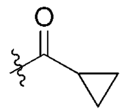 | 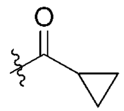 | |
| 74 | F | H | H | F | 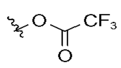 | H | 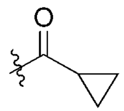 | 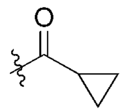 | |
| 75 | H | F | F | H | 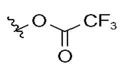 | H | 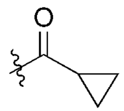 | 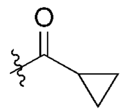 | |
| 76 | H | F | H | F | 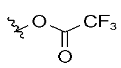 | H | 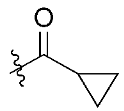 | 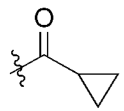 | |
| 77 | H | H | F | F | 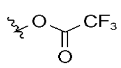 | H | 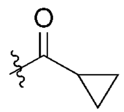 | 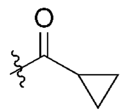 | |
| 78 | F | H | H | H | OH | H | $CH_3$ | $CH_3$ | |
| 79 | H | F | H | H | OH | H | $CH_3$ | $CH_3$ | |
| 80 | H | H | F | H | OH | H | $CH_3$ | $CH_3$ | |
| 81 | H | H | H | F | OH | H | $CH_3$ | $CH_3$ | |

(continued)

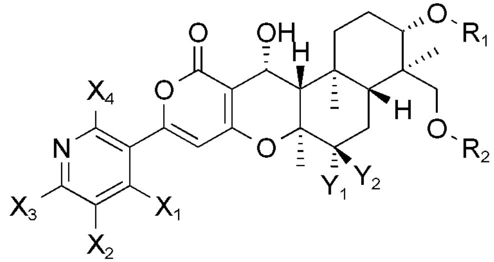

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 82 | F | H | H | H | OH | H | 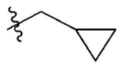 | 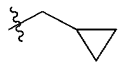 | |
| 83 | H | F | H | H | OH | H | 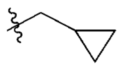 | 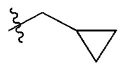 | |
| 84 | H | H | F | H | OH | H | 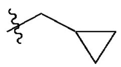 | 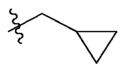 | |
| 85 | H | H | H | F | OH | H | 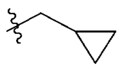 | 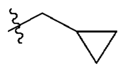 | |
| 86 | F | F | H | H | OH | H | 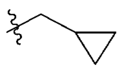 | 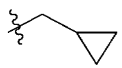 | |
| 87 | F | H | F | H | OH | H | 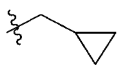 | 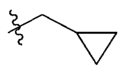 | |
| 88 | F | H | H | F | OH | H | 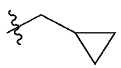 | 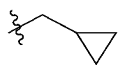 | |
| 89 | H | F | F | H | OH | H | 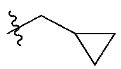 | 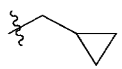 | |
| 90 | H | F | H | F | OH | H | 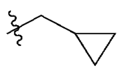 | 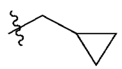 | |

(continued)

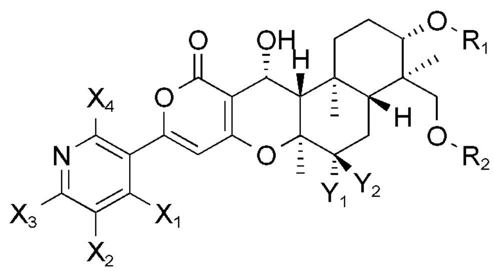

I

| No. | $X_1$ | $X_2$ | $X_3$ | $X_4$ | $Y_1$ | $Y_2$ | $R_1$ | $R_2$ | $^1$H NMR |
|---|---|---|---|---|---|---|---|---|---|
| 91 | H | H | F | F | OH | H | 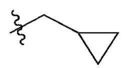 | 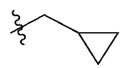 | |
| 92 | F | H | H | H | =O | | $COCH_2CH_3$ | $COCH_2CH_3$ | |
| 93 | F | H | H | H | =O | | 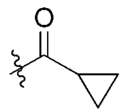 | 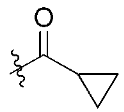 | |
| 94 | H | F | H | H | =O | | $COCH_2CH_3$ | $COCH_2CH_3$ | |
| 95 | H | F | H | H | =O | | 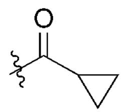 | 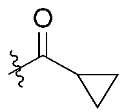 | $^1$H NMR (400 MHz, Chloroform-d) δ 8.85-8.78 (m, 1H), 8.60-8.51 (m, 1H), 7.86-7.78 (m, 1H), 6.73 (s, 1H), 5.05 (s, 1H), 4.85-4.77 (m, 1H), 3.83 (d, J = 11.6 Hz, 1H), 3.65 (d, J = 11.6 Hz, 1H), 2.98-2.86 (m, 1H), 2.77 (t, J = 14.6 Hz, 1H), 2.52 (d, J = 14.6 Hz, 1H), 2.26 (d, J = 14.6 Hz, 1H), 1.96-1.89 (m, 1H), 1.87-1.82 (m, 1H), 1.81-1.80 (m, 2H), 1.77-1.73 (m, 1H), 1.62-1.54 (m, 6H), 1.50-1.40 (m, 1H), 1.33-1.27 (m, 1H), 1.02-0.91 (m, 7H), 0.89-0.79 (m, 4H). |
| 96 | H | H | F | H | =O | | $COCH_2CH_3$ | $COCH_2CH_3$ | |
| 97 | H | H | F | H | =O | | 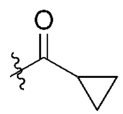 | 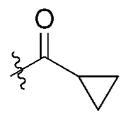 | |
| 98 | H | H | H | F | =O | | $COCH_2CH_3$ | $COCH_2CH_3$ | |

(continued)

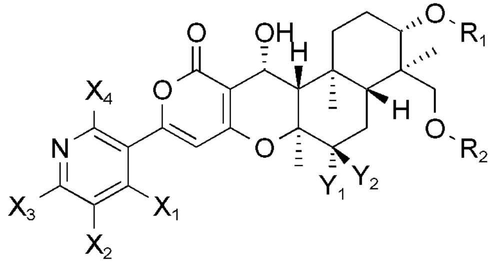

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 99 | H | H | H | F | =O | | 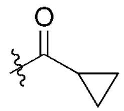 | 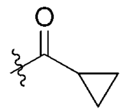 | |
| 100 | F | F | H | H | =O | | 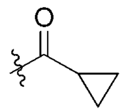 | 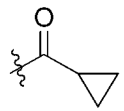 | |
| 101 | F | H | F | H | =O | | 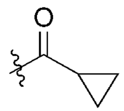 | 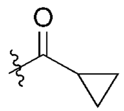 | |
| 102 | F | H | H | F | =O | | 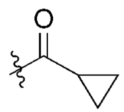 | 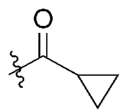 | |
| 103 | H | F | F | H | =O | | 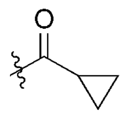 | 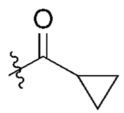 | |
| 104 | H | F | H | F | =O | | 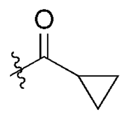 | 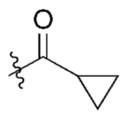 | |

(continued)

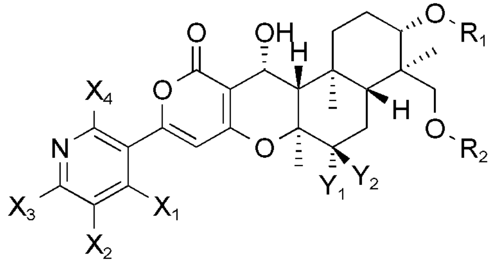

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 105 | H | H | F | F | =O | | 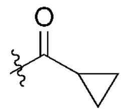 | 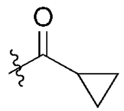 | |
| 106 | F | F | H | H | =O | | $COCH_2CH_3$ | $COCH_2CH_3$ | |
| 107 | F | H | F | H | =O | | $COCH_2CH_3$ | $COCH_2CH_3$ | |
| 108 | F | H | H | F | =O | | $COCH_2CH_3$ | $COCH_2CH_3$ | |
| 109 | H | F | F | H | =O | | $COCH_2CH_3$ | $COCH_2CH_3$ | |
| 110 | H | F | H | F | =O | | $COCH_2CH_3$ | $COCH_2CH_3$ | |
| 111 | H | H | F | F | =O | | $COCH_2CH_3$ | $COCH_2CH_3$ | |
| 112 | F | H | H | H | =O | | $CH_3$ | $CH_3$ | |
| 113 | H | F | H | H | =O | | $CH_3$ | $CH_3$ | |
| 114 | H | H | F | H | =O | | $CH_3$ | $CH_3$ | |
| 115 | H | H | H | F | =O | | $CH_3$ | $CH_3$ | |
| 116 | F | H | H | H | =O | | 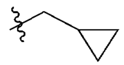 | 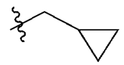 | |
| 117 | H | F | H | H | =O | | 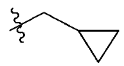 | 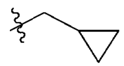 | |
| 118 | H | H | F | H | =O | | 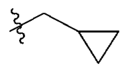 | 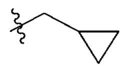 | |

(continued)

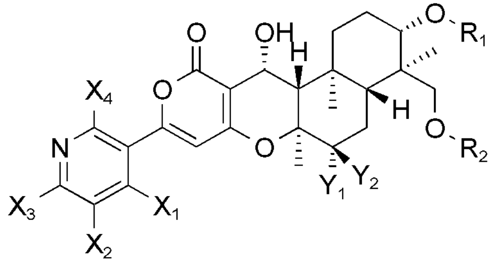

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 119 | H | H | H | F | =O | | 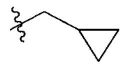 | 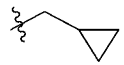 | |
| 120 | F | F | H | H | =O | | 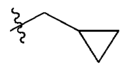 | 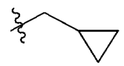 | |
| 121 | F | H | F | H | =O | | 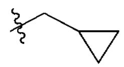 | 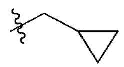 | |
| 122 | F | H | H | F | =O | | 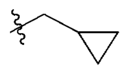 | 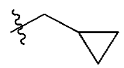 | |
| 123 | H | F | F | H | =O | | 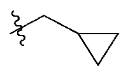 | 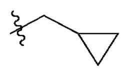 | |
| 124 | H | F | H | F | =O | | 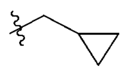 | 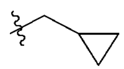 | |
| 125 | H | H | F | F | =O | | 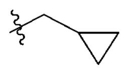 | 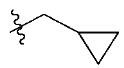 | |
| 126 | H | F | H | H | 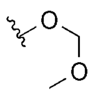 | H | 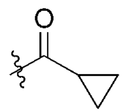 | 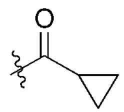 | $^1H$ NMR (300 MHz, Chloroform-d) δ 8.84-8.79 (m, 1H), 8.54 (d, $J$ = 2.6 Hz, 1H), 7.83 (dt, $J$ = 9.1, 2.3 Hz, 1H), 6.45 (s, 1H), 4.97 (d, $J$ = 4.1 Hz, 1H), 4.83 (t, $J$ = 6.2 Hz, 2H), 4.76 (d, $J$= 6.6 Hz, 1H), 4.01 (d, $J$ = 11.7 Hz, 1H), 3.70-3.60 (m, 2H), 3.45 (s, 3H), 2.88 (s, 1H), 2.18-2.07 (m, 1H), 1.93-1.79 (m, 3H), 1.65 (s, 3H), 1.63-1.54 (m, 3H), 1.46-1.34 (m, 6H), 1.04-0.94 (m, 4H), 0.90 (s, 3H), 0.89-0.83 (m, 4H). |

(continued)

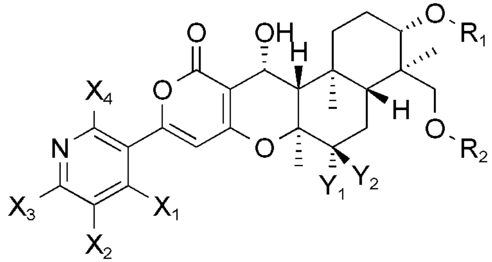

I

| No. | $X_1$ | $X_2$ | $X_3$ | $X_4$ | $Y_1$ | $Y_2$ | $R_1$ | $R_2$ | $^1H$ NMR |
|---|---|---|---|---|---|---|---|---|---|
| 127 | H | F | H | H | OTBS | H | 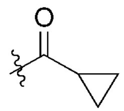 | 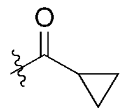 | $^1H$ NMR (300 MHz, Chloroform-*d*) δ 8.84-8.78 (m, 1H), 8.57-8.54 (m, 1H), 7.86-7.79 (m, 1H), 6.37 (s, 1H), 5.00-4.93 (m, 1H), 4.85-4.76 (m, 1H), 3.91 (d, *J* = 11.8 Hz, 1H), 3.72-3.63 (m, 2H), 2.18-2.08 (m, 1H), 1.92-1.75 (m, 3H), 1.67-1.51 (m, 9H), 1.40 (s, 3H), 1.01-0.96 (m, 4H), 0.95 (s, 9H), 0.90 (s, 3H), 0.89-0.84 (m, 4H), 0.15 (s, 3H), 0.11 (s, 3H). |
| 128 | H | F | H | H | OH | H | 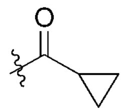 | $COCH_2CH_3$ | $^1H$ NMR (300 MHz, Chloroform-d) δ 8.88-8.80 (m, 1H), 8.50-8.46 (m, 1H), 7.79 (d, *J* = 9.1 Hz, 1H), 6.52 (s, 1H), 4.93 (s, 1H), 4.75 (dt, *J* = 11.0, 5.3 Hz, 1H), 3.89-3.59 (m, 3H), 3.31 (s, 1H), 3.13 (s, 1H), 2.34-2.20 (m, 2H), 2.14-2.03 (m, 1H), 1.90-1.70 (m, 3H), 1.62 (s, 3H), 1.58-1.47 (m, 2H), 1.42-1.33 (m, 5H), 1.33-1.25 (m, 1H), 1.10-1.01 (m, 3H), 0.95-0.88 (m, 2H), 0.85 (s, 3H), 0.83-0.77 (m, 2H). |
| 129 | H | F | H | H | OH | H | 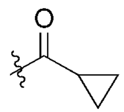 | $CH_2CH_3$ | $^1H$ NMR (300 MHz, Chloroform-d) δ 8.84-8.78 (m, 1H), 8.54 (d, *J* = 2.7 Hz, 1H), 7.87-7.79 (m, 1H), 6.52 (s, 1H), 5.02-4.85 (m, 2H), 3.90-3.79 (m, 1H), 3.44-3.26 (m, 2H), 3.10 (d, *J* = 9.8 Hz, 1H), 2.91 (d, *J* = 9.7 Hz, 2H), 2.14-2.01 (m, 2H), 1.89-1.79 (m, 3H), 1.71-1.51 (m, 8H), 1.38 (s, 3H), 1.11 (t, *J* = 6.9 Hz, 3H), 0.99-0.94 (m, 2H), 0.86-0.77 (m, 5H). |
| 130 | H | F | H | H | OH | $CH_3$ | 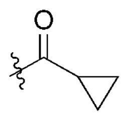 | 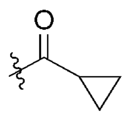 | $^1H$ NMR (300 MHz, Chloroform-d) δ 8.85-8.78 (m, 1H), 8.59-8.50 (m, 1H), 7.87-7.79 (m, 1H), 6.55 (s, 1H), 4.97 (s, 1H), 4.85 (dd, J = 11.2, 5.3 Hz, 1H), 3.91 (d, J = 11.9 Hz, 1H), 3.68 (d, J = 11.9 Hz, 1H), 2.92 (s, 1H), 2.17 (d, J = 13.2 Hz, 1H), 1.91-1.86 (m, 2H), 1.85-1.75 (m, 3H), 1.58-1.52 (m, 5H), 1.44 (s, 3H), 1.40 (s, 3H), 1.33 (s, 3H), 1.00-0.95 (m, 4H), 0.90-0.82 (m, 7H). |

(continued)

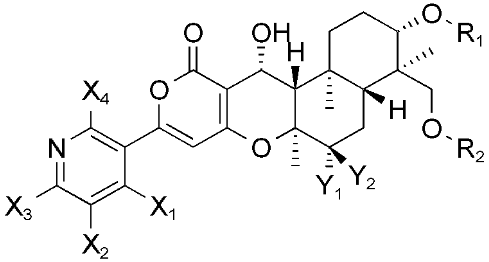

I

| No. | $X_1$ | $X_2$ | $X_3$ | $X_4$ | $Y_1$ | $Y_2$ | $R_1$ | $R_2$ | $^1$H NMR |
|---|---|---|---|---|---|---|---|---|---|
| 131 | H | H | F | H | 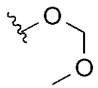 | H | 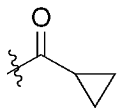 | 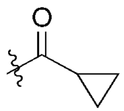 | $^1$H NMR (300 MHz, Chloroform-d) δ 8.64 (s, 1H), 8.22-8.15 (m, 1H), 7.04 (dd, *J* = 8.8, 3.0 Hz, 1H), 6.36 (s, 1H), 5.00-4.93 (m, 1H), 4.87-4.73 (m, 3H), 4.04-3.98 (m, 1H), 3.71-3.61 (m, 2H), 3.45 (s, 3H), 2.85 (s, 1H), 2.18-2.10 (m, 1H), 1.92-1.78 (m, 3H), 1.68-1.60 (m, 6H), 1.45-1.36 (m, 6H), 1.04-0.95 (m, 4H), 0.92-0.82 (m, 7H). |
| 132 | H | H | F | H | OTBS | H | 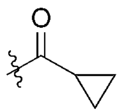 | 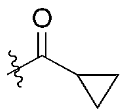 | |
| 133 | H | H | F | H | OH | H | 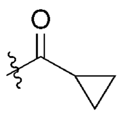 | $COCH_2CH_3$ | $^1$H NMR (400 MHz, Chloroform-*d*) δ 8.63 (s, 1H), 8.22-8.16 (m, 1H), 7.07-7.02 (m, 1H), 6.45 (s, 1H), 4.97 (dd, *J* = 3.0, 1.0 Hz, 1H), 4.81 (dd, *J* = 11.0, 5.5 Hz, 1H), 3.83-3.73 (m, 3H), 2.85 (s, 1H), 2.38-2.27 (m, 2H), 2.17-2.12 (m, 1H), 1.90-1.79 (m, 3H), 1.65 (s, 3H), 1.55-1.49 (m, 2H), 1.47-1.39 (m, 5H), 1.38-1.34 (m, 1H), 1.12 (t, *J* = 7.6 Hz, 3H), 0.99-0.94 (m, 2H), 0.91 (s, 3H), 0.88-0.84 (m, 2H). |
| 134 | H | H | F | H | OH | H | 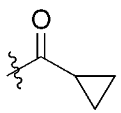 | $CH_2CH_3$ | |
| 135 | H | H | F | H | OH | $CH_3$ | 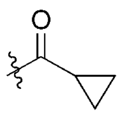 | 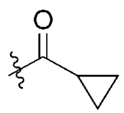 | |

Several methods for preparing the compounds of the present invention are illustrated in detail in the following schemes and examples. Raw materials can be purchased on the market or can be prepared by methods known in literature or as shown in the detailed description. A person skilled in the art should understand that other synthetic routes may also be used to synthesize the compounds of the present invention. Although the specific raw materials and conditions in the synthetic routes have been illustrated below, they may be easily replaced with other similar raw materials and conditions. Various isomers and the like of the compounds resulted from these modifications or variants of the preparation methods of the present invention are all included in the scope of the present invention. In addition, the preparation methods as described below can be further modified according to the disclosure of the present invention using conventional chemical methods well known to a person skilled in the art. For example, the protection for appropriate groups during the reaction, etc.

Examples of methods provided below are used to enhance further understanding of the preparation methods of the present invention, and the specific substances, varieties and conditions employed are determined to be further illustrations of the present invention and not to limit its reasonable scope. Reagents used in the synthesis of the compounds shown in the following tables are either commercially available or can be easily prepared by a person of ordinary skill in the art.

Examples of representative compounds are as follows. The synthesis methods of other compounds are similar and will not be described in detail here.

1. Synthesis of Compound 5 and 38

(1) Fermentation production of Compound 38

*Neosartorya pseudofischeri* was used to carry out fermentation culture with 5-fluoronicotinic acid as a substrate, and then the fermentation broth was filtered to collect the cells. The cells were added with methanol, stirred and filtered. The methanol extract of the cells was collected and concentrated by decompression to obtain an oily concentrate. Purification by normal phase silica gel column chromatography was performed to obtain Compound 38.

(2) Compound 38 (30 mg) was dissolved in methanol (2 mL), added with 30% sodium methoxide solution (72 mg, 8 eq), and stirred overnight at room temperature. When the raw materials were detected by In-Process Control to be consumed completely, the aqueous solution (0.2 mL) of ammonium chloride was subsequently added into the system to quench the reaction. After the reaction solution was spin-dried, the obtained solid was dissolved with ethyl acetate (4 mL) and filtered. The solid obtained by spin-drying the filtrate was washed with a small amount of water to remove the residual ammonium chloride, and Compound 5-2 (23 mg, 97% yield) was obtained by drying the solid.

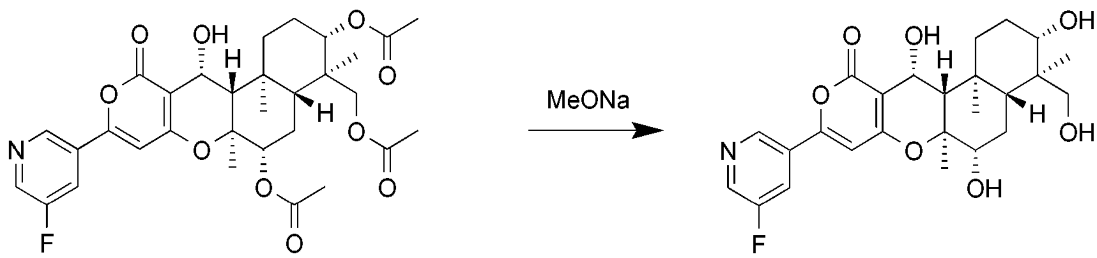


38

5-2

(3) Compound 5-2 (23 mg) was dissolved in dichloromethane (5 mL), sequentially added with 4-dimethylaminopyridine (35 mg, 6 eq), triethylamine (29 mg, 6 eq), cyclopropanecarboxylic acid (58 mg, 14 eq) and 1-(3-dimethylaminopropyl)-3-ethylcarbodiimide hydrochloride (110 mg, 12 eq) at room temperature under stirring. After reaction for 1 h, the completion of the reaction was detected. The system was added with water (2 mL) to separate the liquid. The dichloromethane phase was sequentially washed with 0.05 M hydrochloric acid, sodium bicarbonate, and sodium chloride. The obtained organic phase was spin-dried and purified by normal phase silica gel column chromatography to obtain Compound 5-3 (32 mg, 97% yield).

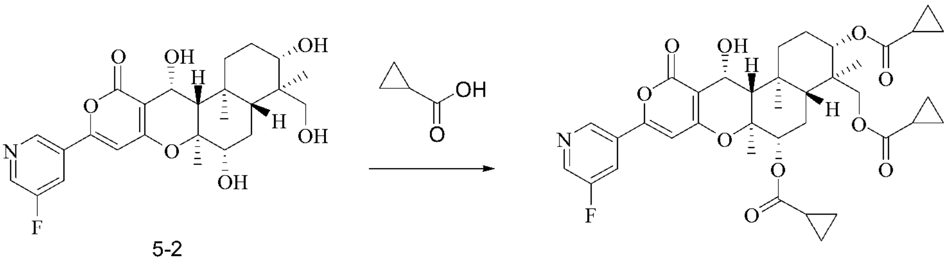

5-2

5-3

(4) Compound 5-3 (32 mg) was dissolved in methanol (5 mL), sequentially added with a small amount of water and 1,8-diaza-bicyclo[5.4.0]undeca-7-ene (8 mg, 1.1 eq), and reacted for 2 h at room temperature under stirring. When the raw materials were detected by In-Process Control to be consumed completely, purification by normal phase silica gel column chromatography was performed to obtain Compound 5 (13 mg, 45% yield).

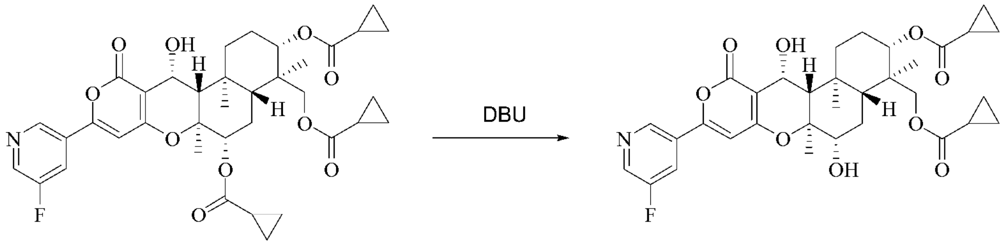


5-3

5

2. Synthesis of Compound 9

(1) In ice bath, TMEDA (0.1 eq) was added into a three-necked flask under nitrogen protection, added dropwise with LHMDS (5 eq), and reacted for 10 min at a constant temperature. The system was added to with THF solution of Compound 9-1 (1 g), reacted for half an hour at a constant temperature and then warmed to room temperature and reacted for 1 h. In ice bath, THF solution of Compound 9-2 (3 eq) was added to the system and reacted for 1 hour at a constant temperature. Upon the completion of the reaction, the reaction solution was poured into aqueous solution of ammonium chloride and extracted with ethyl acetate. The organic phase was dried, concentrated, and purified through normal phase chromatography to obtain 430 mg of Compound 9-3 with a yield of 34%.

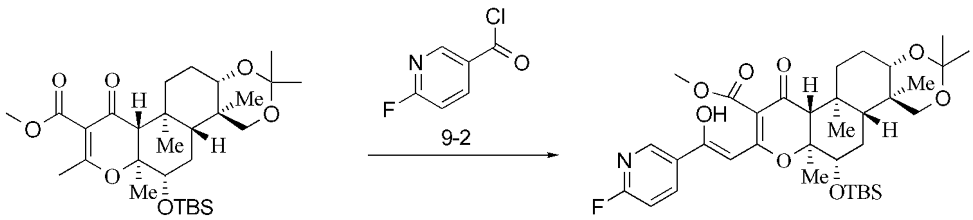

9-2

9-1

9-3

(2) Under a negative pressure of 0mmHg, Compound 9-3 (430 mg) reacted for 15 min in oil bath at 150°C to obtain the crude product of Compound 9-4, which was directly used in the next step.

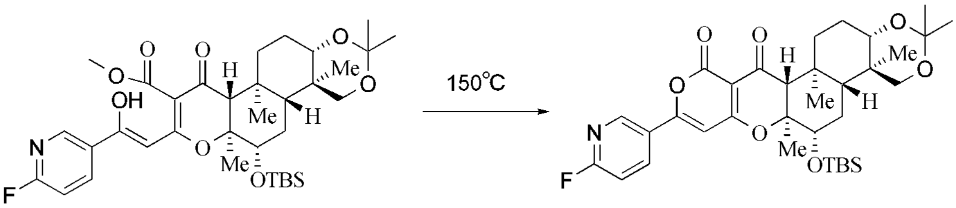


9-3

9-4

(3) The crude product of Compound 9-4 was dissolved in 10 mL of THF, added with 10 mL of 80% acetic acid solution, and stirred at room temperature. After the raw materials were consumed completely, the reaction solution was poured into saturated sodium bicarbonate solution in ice bath and extracted with ethyl acetate. The organic phase was dried and concentrated to obtain 380 mg of the crude product of Compound 9-5 with a crude yield of 99%.

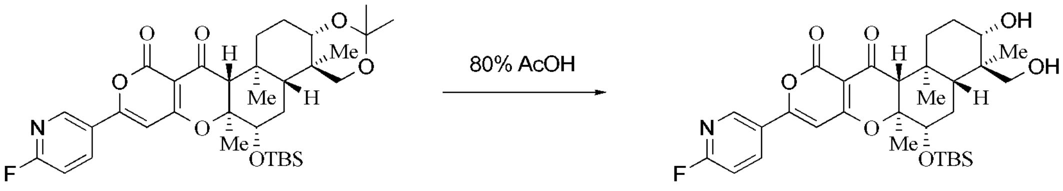


9-4 9-5

(4) The crude product of Compound 9-5 (380 mg), DMAP (6 eq), EDCI (4 eq), and cyclopropanecarboxylic acid (5 eq) were sequentially added in 10 mL of DCM and reacted at room temperature. After the reaction, water was added for extraction. The organic phase was washed once with dilute hydrochloric acid, washed once with saturated sodium bicarbonate solution, washed once with sodium chloride solution, dried, and concentrated to obtain 460 mg of the crude product of Compound 9-6 with a crude yield of 98%.

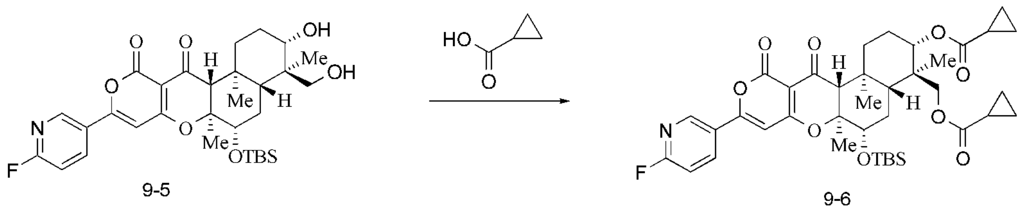

9-5 9-6

(5) The crude product of Compound 9-6 (460 mg) was dissolved in 10 mL of THF, and in ice bath, added with 3 mL of pyridine and 2 mL of hydrogen fluoride-pyridine solution. After the reaction, the reaction solution was poured into ice water for extraction. The organic phase was washed with saturated sodium bicarbonate solution, dried, and concentrated to obtain 240 mg of the crude product of Compound 9-7 with a crude yield of 62%.

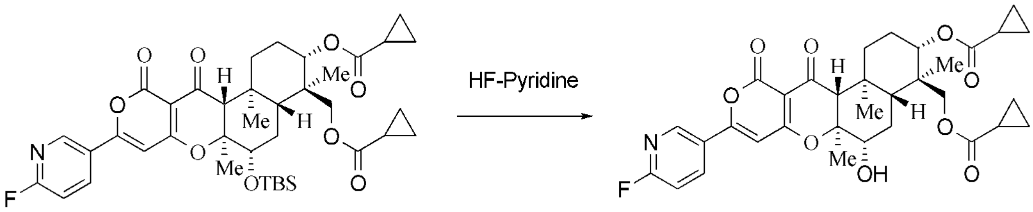


9-6 9-7

(6) The crude product of Compound 9-7 (240 mg) was dissolved in 10 mL of THF, sequentially added with cerium trichloride heptahydrate (1.2 eq) and sodium borohydride (2.4 eq), and reacted at room temperature. After the reaction, the reaction solution was filtered using diatomaceous earth. The filtrate was stirred with silica gel and purified through normal phase chromatography to obtain 60 mg of Compound 9 (pale yellow solid, 25% yield).

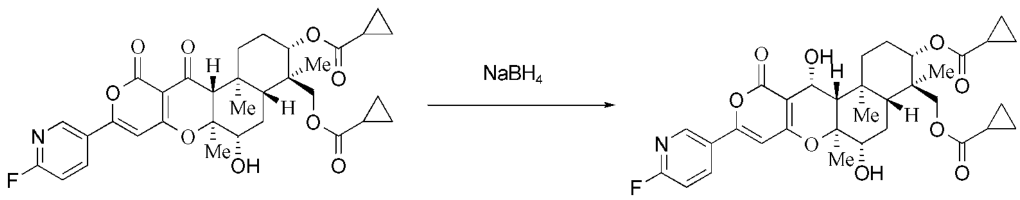


9-7 9

3. Synthesis of Compound 9

(1) Compound 9-6 (950.0 mg, 1.62 mmol) was dissolved in 5 mL of dichloromethane, and in ice-water bath,

sequentially added with triethylamine (TEA, 1.32 g, 12.96 mmol), cyclopropanecarboxylic acid (696.6 mg, 8.10 mmol), 4-dimethylaminopyridine (DMAP, 395.3 mg, 3.24 mmol), and 1-ethyl-3-(3'-dimethylaminopropyl)carbodiimide hydrochloride (EDCI, 1.55 g, 8.10 mmol), and stirred at room temperature for 30 min. The LCMS indicated the completion of the reaction. The reaction solution was diluted by adding water and dichloromethane. The organic phase was sequentially washed with saturated ammonium chloride solution and saturated saline solution, dried, and concentrated to obtain Compound 9-8 (600.0 mg, 51% yield, pale yellow solid).

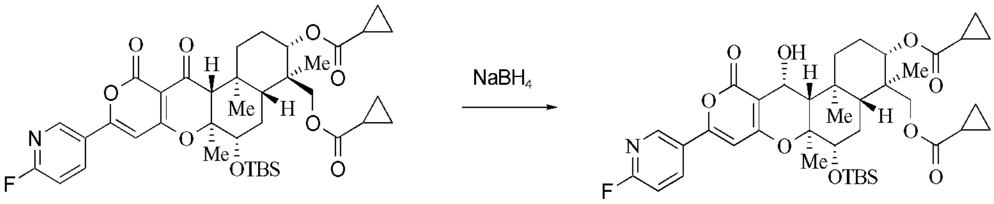


9-6 9-8

(2) Compound 9-8 (300.0 mg, 0.41 mmol) was dissolved in a mixed solvent of pyridine/tetrahydrofuran (2 mL/10 mL), and at 0°C added dropwise with pyridine hydrofluoride (5 mL), and stirred at room temperature for 12 h. The LCMS detected the completion of the reaction. The reaction solution was quenched by saturated aqueous solution of sodium bicarbonate. The aqueous phase was extracted with ethyl acetate. The organic phase was washed with saturated saline solution, dried, and concentrated. The residue was purified through column chromatography (EA/PE = 30-60%) to obtain Compound 9 (150.0 mg, 59.6 mmol, 60% yield, white solid).

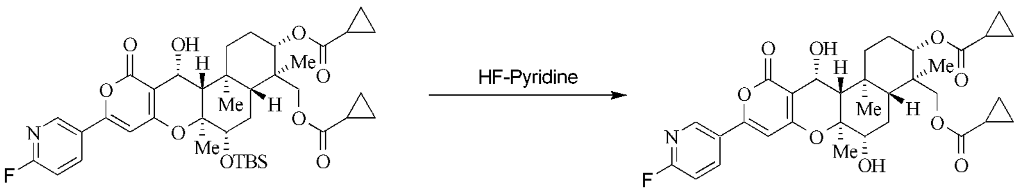


9-8 9

4. Synthesis of Compound 43

Compound 9 (20 mg, 0.03 mmol) was dissolved in 1 mL of dichloromethane, added with triethylamine (32 mg, 0.06 mmol), acetic anhydride (6.1 mg, 0.06 mmol), and 4-dimethylaminopyridine (2 mg, 0.03 mmol), and reacted at room temperature for 0.5 h. The LCMS detected the completion of the reaction. The reaction solution was quenched by aqueous solution of sodium bicarbonate and extracted with ethyl acetate. The organic phase was washed with saturated saline solution, dried, and concentrated. The residue was purified through column chromatography (EA/PE = 1/2) to obtain Compound 43 (10 mg, 47% yield, pale yellow solid).

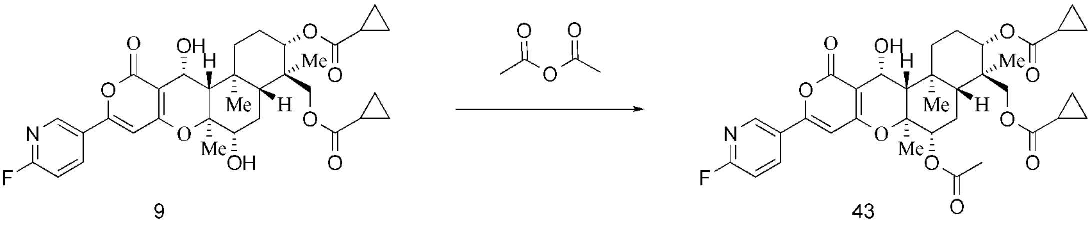

9 43

5. Synthesis of Compound 61

Compound 5 (100 mg, 0.16 mmol) was dissolved in 1 mL of DMF, added with triethylamine (32 mg, 0.32 mmol), Compound 61-1 (67 mg, 0.32 mmol), and 4-dimethylaminopyridine (2 mg, 0.16 mmol), and reacted at room temperature for 0.5 h. The LCMS detected the completion of the reaction. The reaction solution was quenched by aqueous solution of sodium bicarbonate and extracted with ethyl acetate. The organic phase was washed with saturated saline solution, dried, and concentrated. The residue was purified through column chromatography (MeOH:DCM = 1:15) to obtain Compound 61 (10 mg, 9% yield, pale yellow solid).

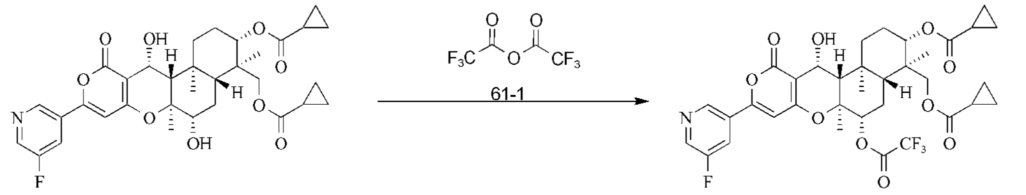


5

61

6. Synthesis of Compound 95

Compound 5 (0.50 g, 0.82 mmol) was added into a 100-mL three-necked flask and dissolved with 5 mL of dichloromethane. The system was placed in ice-salt bath and slowly added with Dess-Martin periodinane (0.52 g, 1.22 mmol) while the temperature was kept no higher than 5°C. Subsequently, the system was reacted at room temperature for 4 h. After the completion of the reaction was detected by LCMS, the reaction solution was added with a small amount of saturated sodium thiosulfate solution to quench the reaction and extracted three times with dichloromethane. The organic phase was dried over anhydrous sodium sulfate and concentrated. The residue was purified through column chromatography (PE:EA = 1:1) to obtain Compound 95 (0.11 g, 22% yield, yellow solid).

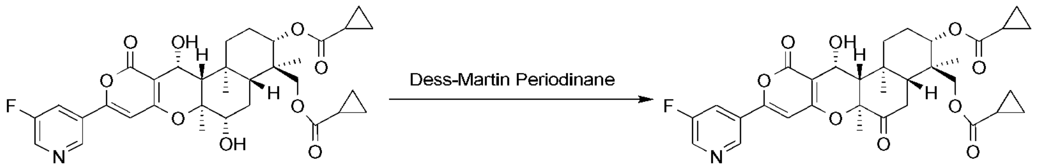


5

95

7. Synthesis of Compound 126

(1) Compound 126-1 was prepared with reference to Compound 9-7. Compound 126-1 (300 mg, 0.48 mmol) was dissolved in 2 mL of 1,2-dichloroethane, sequentially added with bromomethyl methyl ether (306 mg, 2.46 mmol), TBAI (48 mg, 0.39 mmol), and DIEA (906 mg, 2.46 mmol), and reacted at room temperature for 12 h. The LCMS detected the completion of the reaction. The reaction solution was diluted by water and dichloromethane. The organic phase was washed with saturated saline solution, dried, and concentrated. The residue was purified through column chromatography (EA:PE = 1:5) to obtain Compound 126-2 (150 mg, 47% yield, pale yellow solid).

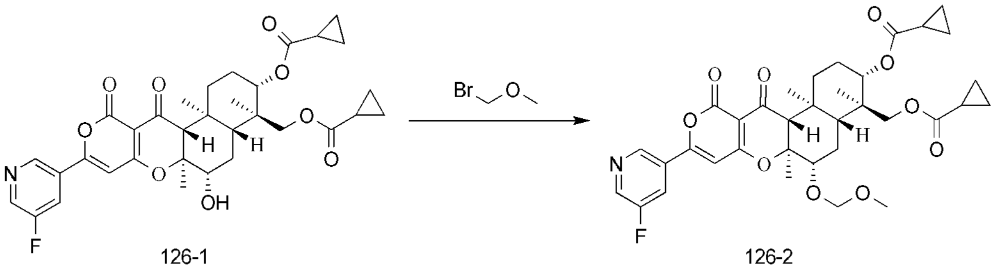

126-1

126-2

(2) Compound 126-2 (150 mg, 0.23 mmol) was dissolved in 2 mL of anhydrous ethanol, and in ice-water bath, sequentially added with $CeCl_3 \cdot 7H_2O$ (85 mg, 0.23 mmol) and $NaBH_4$ (16 mg, 0.46 mmol), and reacted at room temperature for 30 min. The LCMS detected the completion of the reaction. The reaction solution was quenched by aqueous solution of saturated $NaHCO_3$ and extracted with ethyl acetate. The organic phase was washed with water and saturated saline solution, dried, and concentrated. The residue was purified through column chromatography (EA:PE = 1:1) to obtain Compound 126 (59 mg, 38% yield, pale yellow solid).

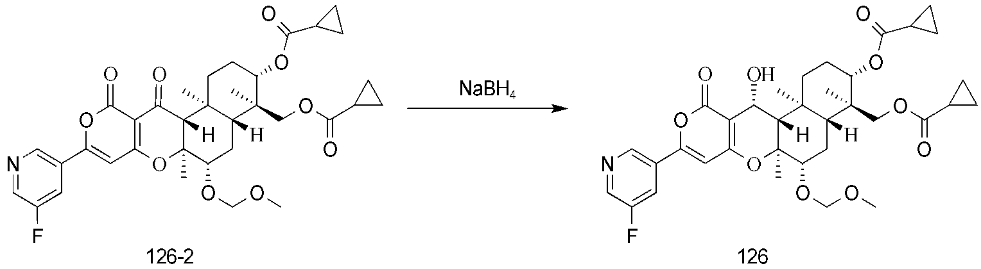


126-2 126

8. Synthesis of Compound 128

(1) Compound 5-2 (25.0 g of crude product) was dissolved in 200 mL of DMF, and in ice-water bath, added with Compound 128-1 (64.3 mL, 526.3 mmol) and pyridinium p-toluenesulfonate (6.6 g, 26.3 mmol), and reacted at room temperature for 30 min. The LCMS detected the completion of the reaction. The reaction solution was quenched by adding aqueous solution of saturated sodium bicarbonate and extracted with dichloromethane. The organic phase was washed with water and saturated saline solution, dried, and concentrated to obtain the crude product which was slurred with PE:EA=10:1. The filter cake was collected and vacuum-dried to obtain the crude product of Compound 128-2 (28.0 g, pale yellow solid) which was directly used in the next step.

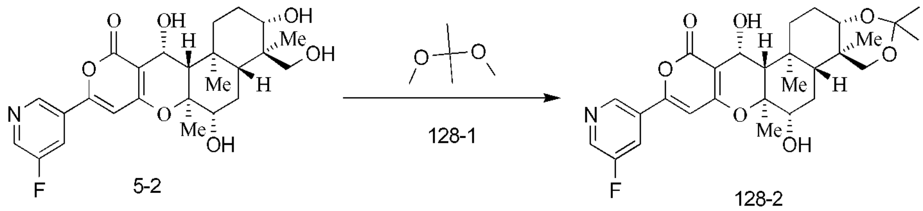


5-2 128-2

(2) Compound 128-2 (28.0 g, crude product) was dissolved in 150 mL of DMF, and in ice-water bath, sequentially added with tert-butyldimethylsilyl chloride (TBSCl, 16.4 g, 108.8 mmol) and imidazole (7.4 g, 108.8 mmol), and stirred at room temperature for 12 h. The LCMS detected the completion of the reaction. The reaction solution was quenched by adding aqueous solution of saturated ammonium chloride and extracted with dichloromethane. The organic phase was washed with water and saturated saline solution, dried, and concentrated to obtain the crude product which was slurred with petroleum ether. The filter cake was collected and vacuum-dried to obtain the crude product of Compound 128-3 (28 g, pale yellow solid) which was directly used in the next step.

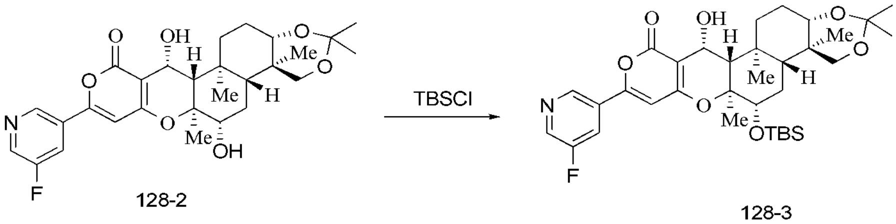


128-2 128-3

(3) Compound 128-3 (28.0 g, crude product) was dissolved in 100 mL of THF, and in ice-water bath, added with 1500 mL aqueous solution of 80% AcOH, and stirred at room temperature for 24 h. The LCMS detected the completion of the reaction. The pH of the reaction solution was adjusted to 7-8 using aqueous solution of saturated sodium bicarbonate. The aqueous phase was extracted with ethyl acetate. The organic phase was washed with saturated saline solution, dried, and concentrated. The residue was purified through column chromatography (EA:PE = 1:5-3:5) to obtain Compound 128-4 (21.0 g, 35.6 mmol, pale yellow solid).

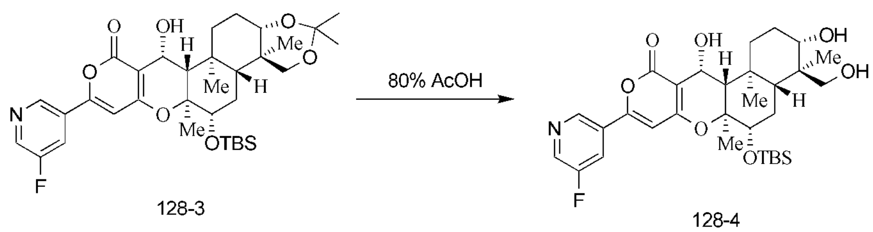


128-3 128-4

(4) Compound 128-4 (500 mg, 0.85 mmol) was dissolved in 2 mL of dichloromethane, added with 4-dimethylaminopyridine (104 mg, 0.85 mmol) and Compound 128-5 (110 mg, 0.85 mmol), and reacted at room temperature for 10 h. The LCMS detected the completion of the reaction. The reaction solution was quenched by aqueous solution of sodium bicarbonate and extracted with dichloromethane. The organic phase was washed with water and saturated saline solution, dried, and concentrated. The residue was purified through column chromatography (THF:PE = 1:4) to obtain Compound 128-6 (500 mg, 91% yield, pale yellow solid).

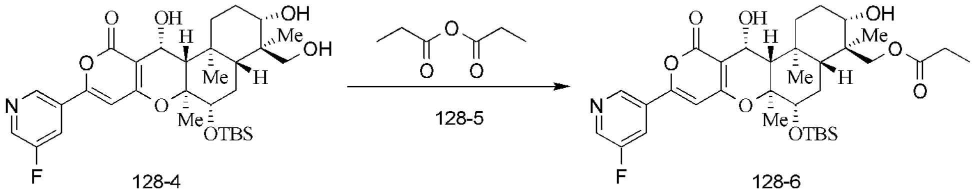


128-4 128-6

(5) Compound 128-6 (500 mg, 0.77 mmol) was dissolved in 2 mL of dichloromethane, at room temperature added with triethylamine (623 mg, 6.16 mmol), cyclopropanecarboxylic acid (265 mg, 3.08 mmol), EDCI (590 mg, 3.08 mmol) and 4-dimethylaminopyridine (188 mg, 1.54 mmol), and reacted for 2 h. The LCMS detected the completion of the reaction. The reaction solution was diluted by water and dichloromethane. The organic phase was washed with saturated ammonium chloride and saturated saline solution, dried, and concentrated. The residue was purified through column chromatography (THF/PE = 1/1) to obtain Compound 128-7 (450 mg, 82% yield, pale yellow solid).

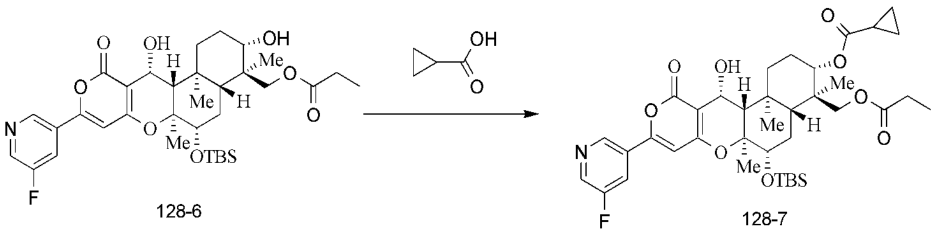

128-6 128-7

(6) Compound 128-7 (450 mg, 0.63 mmol) was dissolved in 1 mL of tetrahydrofuran, and in ice-water bath, added with pyridine (0.5 mL, 6.3 mmol) and hydrofluoric acid/pyridine (0.5 mL, 5.0 mmol), and reacted at room temperature for 12 h. The LCMS detected the completion of the reaction. The reaction solution was quenched by aqueous solution of sodium bicarbonate and extracted with ethyl acetate. The organic phase was washed with saturated saline solution, dried, and concentrated. The residue was purified through column chromatography (MeOH:DCM = 1:20) to obtain Compound 128 (49 mg, 13% yield, pale yellow solid).

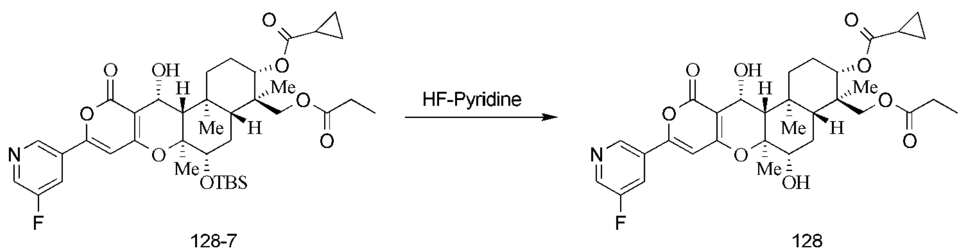


128-7 128

9. Synthesis of Compound 5

(1) Compound 128-4 (21.0 g, 35.6 mmol) was dissolved in 150 mL of DCM, and in ice-water bath, sequentially added with triethylamine (TEA, 28.7 g, 284.8 mmol), cyclopropanecarboxylic acid (15.3 g, 178.0 mmol), 4-dimethylaminopyridine (DMAP, 8.7 g, 71.2 mmol) and 1-ethyl-3-(3'-dimethylaminopropyl)carbodiimide hydrochloride (EDCI, 27.2 g, 142.4 mmol), and reacted at room temperature for 12 h. The LCMS indicated the completion of the reaction. The reaction solution was diluted by adding water and dichloromethane. The organic phase was washed with saturated ammonium chloride solution and saturated saline solution, dried, and concentrated. The residue was purified through column chromatography (EA/PE = 10-50%) to obtain Compound 5-4 (17.5 g, 24.1 mmol, white solid).

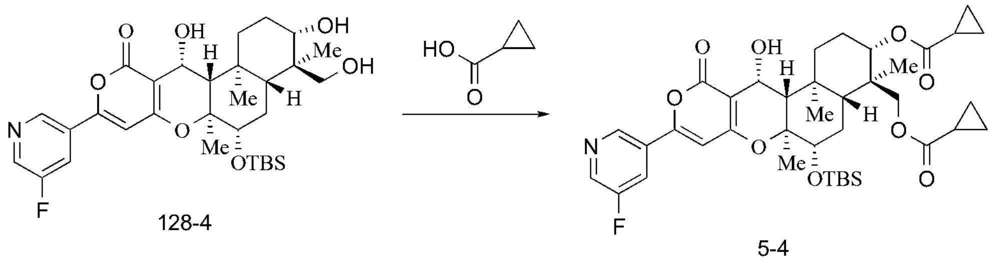

128-4 5-4

(2) Compound 5-4 (17.5 g, 24.1 mmol) was dissolved in a mixed solvent of pyridine/tetrahydrofuran (20 mL/100 mL), and at 0°C added dropwise with pyridine hydrofluoride (30 mL), and stirred at room temperature for 24 h. The LCMS detected the completion of the reaction. The reaction solution was quenched by aqueous solution of saturated sodium bicarbonate. The aqueous phase was extracted with ethyl acetate. The organic phase was washed with water and saturated saline solution, dried, and concentrated. The residue was purified through column chromatography (EA/PE = 30-60%) to obtain Compound 5 (13.4 g, 21.9 mmol, white solid).

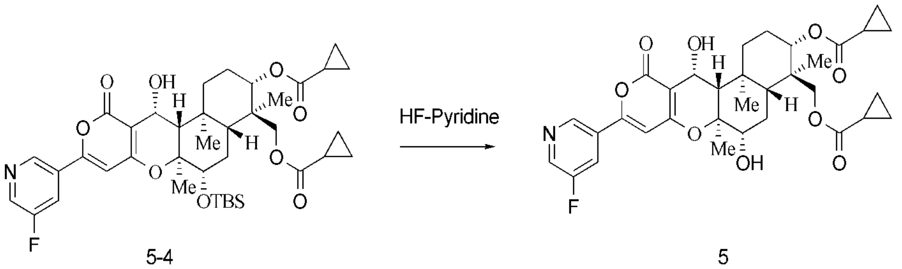


5-4 5

10. Synthesis of Compound 131

(1) Compound 9-7 (120.0 mg, 0.25 mmol) was dissolved in 2 mL of 1,2-dichloroethane, sequentially added with bromomethyl methyl ether (31.0 mg, 0.25 mmol), TBAI (11.0 mg, 0.03 mmol), and DIEA (32.3 mg, 0.25 mmol), and reacted at room temperature for 3 h. The LCMS detected the completion of the reaction. The reaction solution was diluted by water and dichloromethane. The organic phase was washed with saturated saline solution, dried, and concentrated. The residue was purified through column chromatography (EA/PE = 1/5) to obtain Compound 131-1 (30.0 mg, 47% yield, pale yellow solid).

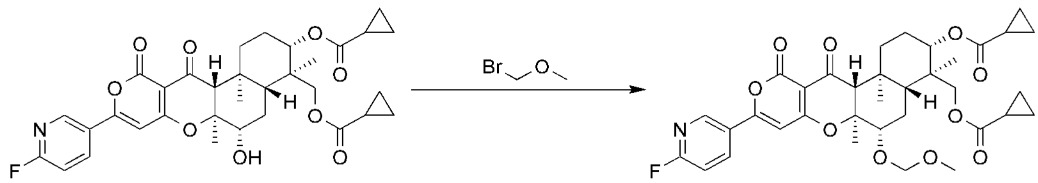

9-7 131-1

(2) Compound 131-1 (30.0 mg, 0.05 mmol) was dissolved in 1 mL of tetrahydrofuran, and in ice-water bath, sequentially added with $CeCl_3 \cdot 7H_2O$ (18.6 mg, 0.05 mmol) and $NaBH_4$ (3.8 mg, 0.10 mmol), and reacted at room temperature for 30 min. The LCMS detected the completion of the reaction. The reaction solution was quenched by aqueous solution of saturated $NaHCO_3$ and extracted with ethyl acetate. The organic phase was washed with water and saturated saline solution, dried, and concentrated. The residue was purified through column chromatography (EA/PE = 1/1) to obtain Compound 131 (8.0 mg, 27% yield, pale yellow solid).

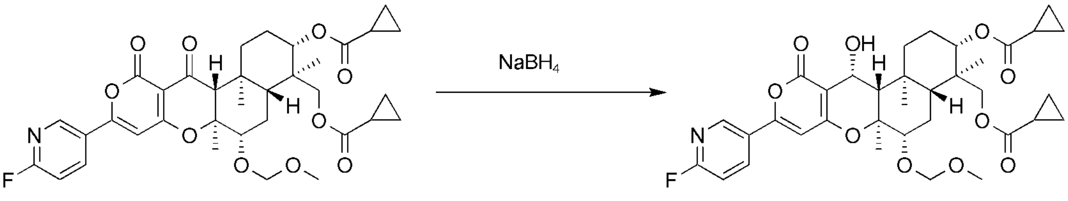


131-1 131

11. Synthesis of Compound 133

(1) Compound 9-5 (350 mg, 0.60 mmol) was dissolved in 2 mL of dichloromethane, added with 4-dimethylaminopyridine (77.5 mg, 0.60 mmol) and propionic anhydride (72.8 mg, 0.60 mmol), and reacted at room temperature for 30 min. The LCMS detected the completion of the reaction. The reaction solution was quenched by aqueous solution of sodium bicarbonate and extracted with dichloromethane. The organic phase was washed with saturated saline solution, dried, and concentrated. The residue was purified through column chromatography (THF:PE = 1:4) to obtain Compound 133-1 (140 mg, 37% yield, pale yellow solid).

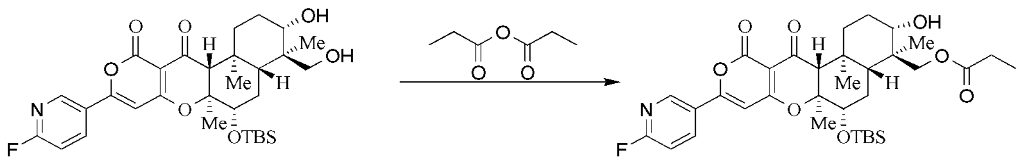

9-5 133-1

(2) Compound 133-1 (140 mg, 0.22 mmol) was dissolved in 2 mL of dichloromethane, at room temperature added with triethylamine (175 mg, 1.74 mmol), cyclopropanecarboxylic acid (73.5 mg, 0.87 mmol), EDCI (164.5 mg, 0.87 mmol) and 4-dimethylaminopyridine (52.5 mg, 0.43 mmol), and reacted at room temperature for 2 h. The LCMS detected the completion of the reaction. The reaction solution was diluted by water and dichloromethane. The organic phase was washed with saturated ammonium chloride and saturated saline solution, dried, and concentrated. The residue was purified through column chromatography (THF/PE = 1/3) to obtain Compound 133-2 (80 mg, 52% yield, pale yellow solid).

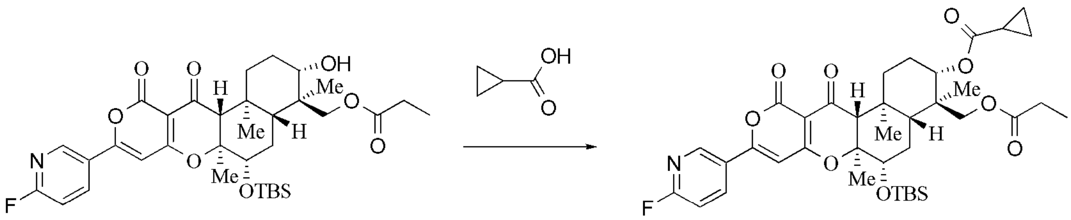

133-1 133-2

(3) Compound 133-2 (80 mg, 0.63 mmol) was dissolved in 1 mL of tetrahydrofuran, and in ice-water bath, added with pyridine (0.5 mL, 6.3 mmol) and hydrofluoric acid/pyridine (0.5 mL, 5.0 mmol), and reacted at room temperature for 12 h. The LCMS detected the completion of the reaction. The reaction solution was quenched by aqueous solution of sodium bicarbonate and extracted with ethyl acetate. The organic phase was washed with saturated saline solution, dried, and concentrated. The residue was purified through column chromatography (THF/PE = 1/1) to obtain Compound 133-3 (50 mg, 75% yield, pale yellow solid).

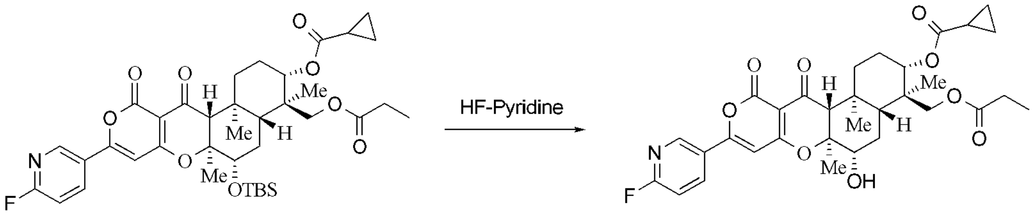


133-2 133-3

(2) Compound 133-3 (50 mg, 0.08 mmol) was dissolved in 1 mL of tetrahydrofuran, and in ice-water bath, added with $CeCl_3 \cdot 7H_2O$ (30 mg, 0.08 mmol) and $NaBH_4$ (6.5 mg, 0.17 mmol), and reacted at room temperature for 30 min. The LCMS detected the completion of the reaction. The reaction solution was quenched by aqueous solution of sodium bicarbonate and extracted with ethyl acetate. The organic phase was washed with saturated saline solution, dried, and concentrated. The residue was purified through column chromatography (THF/PE = 1/1) to obtain Compound 133 (30 mg, 40% yield, pale yellow solid).

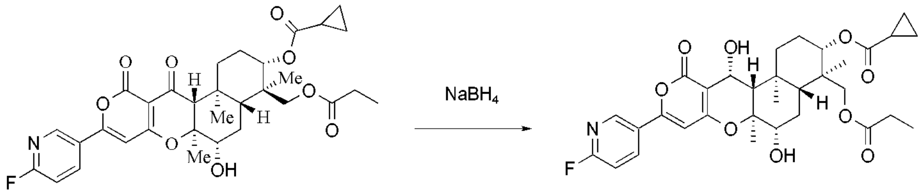


133-3 133

Detailed Embodiments of the Invention

Evaluation of Biological Activity:

(1) Insecticidal activity assay:

1.1) Spray treatment test

After the technical material was dissolved with acetone, the drug solution was diluted to gradient doses with distilled water. *Myzus* persicae-bearing radish sprout pot plants of the same larval age and seedling age were selected, equally sprayed on the front and back throughly and evenly using a handheld spraying equipment. Each pot of radish was sprayed 8-10 times, with water as the control, and the procedure was repeated 3 times. After drug application, the pots were transferred to culture conditions. The 3rd instar *Apolygus lucorum* test insects of the same physiological state reared indoor were selected and put into disposable clear plastic cups. Each cup was inoculated with 20 test insects and put into with 4 grains of fruit corn kernels, and then sprayed using a spray tower. After spraying, the cups were covered with cup lids. The procedure was repeated 3 times, with water as the control. After drug application, the insects were transferred to rearing conditions. *Rhopalosiphum* padi-bearing wheat seedling pot plants of the same larval age and seedling age were selected, equally sprayed on the front and back throughly and evenly using a handheld spraying equipment. Each pot of wheat was sprayed 8-10 times, with water as the control, and the procedure was repeated 3 times. After drug application, the plants were transferred to culture conditions. The results were investigated after n days and the death number per treatment was collected, respectively. The mortality was calculated as: Mortality (%) = Number of dead insects / Number of test insects) × 100.

Table 2 Results of insecticidal tests

| No. | *Myzus persicae* 6DAA | | *Rhopalosiphum padi* 6DAA | | *Apolygus lucorum* 3DAA | |
|---|---|---|---|---|---|---|
| | Mortality /% | Drug concentration /ppm | Mortality /% | Drug concentration /ppm | Mortality /% | Drug concentration /ppm |
| 5 | 90 | 10 | 95 | 60 | 64 | 30 |
| 9 | 90 | 10 | 98 | 60 | 66 | 30 |
| 40 | N | N | 90 | 60 | N | N |
| 61 | 75 | 10 | 80 | 60 | N | N |
| 95 | 80 | 10 | 90 | 60 | N | N |
| 126 | 80 | 10 | N | N | N | N |
| 128 | 80 | 10 | 85 | 60 | N | N |
| 131 | 85 | 10 | 80 | 60 | N | N |
| 133 | N | N | 55 | 60 | N | N |

(continued)

| No. | *Myzus persicae* 6DAA | | *Rhopalosiphum padi* 6DAA | | *Apolygus lucorum* 3DAA | |
|---|---|---|---|---|---|---|
| | Mortality /% | Drug concentration /ppm | Mortality /% | Drug concentration /ppm | Mortality /% | Drug concentration /ppm |
| Control compound A | 19 | 10 | 26 | 60 | 22 | 30 |
| Note: N represents no data; Control Compound A: | | | | | | |

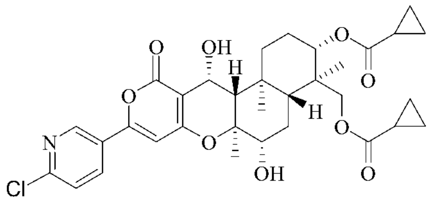
.

1.2) Experiment of acropetal translocation capacity test

Insect-bearing radish sprout plants planted in disposable clear plastic cups were selected, three radish sprouts per cup, and went through 24-h drought treatment in advance prior to experimentation; the radish sprouts were poured from the roots with 20 mL of the formulated drug solution. Each treatment was carried out in triplicate and a treatment with water was employed as the control. Normal light and culture conditions were provided. The results were investigated 6 days after drug treatment. The death number per treatment was collected, respectively. The mortality was calculated, and the representative test results are as shown in Table 3.

$$\text{Mortality} = (\text{Number of dead insects} / \text{Number of test insects}) \times 100\%$$

Table 3 Experimental results of acropetal translocation capacity test

| No. | *Myzus persicae* 6DAA | | *Aphis craccivora* 6DAA | |
|---|---|---|---|---|
| | Mortality/% | Drug concentration/ppm | Mortality/% | Drug concentration/ppm |
| 5 | 85 | 50 | 35 | 25 |
| 9 | 80 | 50 | 30 | 25 |
| Control compound B | 20 | 50 | 0 | 25 |
| Note: Control compound B: Afidopyropen. | | | | |

(2) Insecticidal activity assay of compositions:

2.1) Experimental conditions and operation procedures

Test targets: *Myzus persicae, Bemisia tabaci*

*Myzus persicae:* After the technical material was dissolved with acetone, the drug solution was diluted to gradient doses with distilled water. *Myzus* persicae-bearing radish sprout pot plants of the same larval age and seedling age were selected, equally sprayed on the front and back throughly and evenly using a handheld spraying equipment. Each pot of radish was sprayed 8-10 times, with water as the control, and the procedure was repeated 3 times. After drug application, the pots were transferred to culture conditions. The results were investigated after 6 days and the death number per treatment was collected, respectively. The mortality was calculated as: Mortality (%) = Number of dead insects / Number of test insects) × 100.

*Bemisia tabaci:* After the technical material was dissolved with acetone, the drug solution was diluted to gradient doses with distilled water. A one-liter plastic cup with a lid was used as a test vessel. Two true leaves of a 'Beibei' pumpkin *(Cucurbita maxima* cultivar) were cut off and inserted on a plastic sponge. An appropriate amount of water was dropped

into the sponge to keep the pumpkin seedlings moisturized, and the treated pumpkin seedlings were put into the plastic cup to create a test device. About 200 adult *Bemisia tabaci* were aspirated and put into the test device. The prepared drug solution was evenly sprayed through the upper hole using a handheld electric sprayer. Each treatment was repeated 3 times. Upon the completion of the drug application, the test devices were placed in a process chamber at 25°C with a humidity of 60%. The results were investigated 3 days later. The death number per treatment was collected, respectively. The mortality was calculated as: Mortality (%) = Number of dead insects / Number of test insects) × 100.

2.2) Qualitative evaluation of synergism

Different ratios within the selected ratio range were set for toxicity determination, and the optimal ratio was selected according to synergism. When the synergism > 0, it indicated a synergistic effect; when the synergism was close to 0, it indicated an addition effect; when the synergism < 0, it indicated an antagonistic effect.

$$\text{Synergism} = \text{Actual death rate} - \text{Theoretical death rate}$$

$$\text{Theoretical death rate} = 1 - (1 - P_1)(1 - P_2)$$

wherein, $P_1$, $P_2$-Death rate by each individual agent in a mixed formulation.

Table 4 Qualitative evaluation results of synergisms of compositions

| A+B | A+B Dosage (g a.i./ha) | A:B | Mortality by individual A (%) | Mortality by individual B (%) | Mortality by mixed formulation (%) | Theoretical mortality (%) | Synergistic effect (%) | Target of Action |
|---|---|---|---|---|---|---|---|---|
| 5+Chlorpyrifos | 7.5+300 | 1:40 | 50.8 | 38.7 | 85.6 | 69.8 | 15.8 | *Myzus persicae* |
| 5+Acephate | 7.5+450 | 1:60 | 50.8 | 32.6 | 83.2 | 66.8 | 16.4 | *Myzus persicae* |
| 5+Fipronil | 7.5+7.5 | 1:1 | 50.8 | 33.6 | 82.3 | 67.3 | 15.0 | *Myzus persicae* |
| 5+Lambda-Cyhalothrin | 7.5+ 7.5 | 1:1 | 50.8 | 43.8 | 88.9 | 72.3 | 16.6 | *Myzus persicae* |
| 5+Bifenthrin | 7.5+22.5 | 1:3 | 50.8 | 40.9 | 86.7 | 70.9 | 15.8 | *Myzus persicae* |
| 5+Deltamethrin | 7.5+7.5 | 1:1 | 50.8 | 32.8 | 85.5 | 66.9 | 18.6 | *Myzus persicae* |
| 5+Beta-Cypermethrin | 7.5+15 | 1:2 | 50.8 | 50.3 | 95.5 | 75.5 | 20.0 | *Myzus persicae* |
| 5+Alpha-Cypermethrin | 7.5+7.5 | 1:1 | 50.8 | 30.2 | 83.1 | 65.7 | 17.4 | *Myzus persicae* |
| 5+Imidacloprid | 7.5+15 | 1:2 | 50.8 | 47.6 | 93.5 | 74.2 | 19.3 | *Myzus persicae* |

(continued)

| A+B | A+B Dosage (g a.i./ha) | A:B | Mortality by individual A (%) | Mortality by individual B (%) | Mortality by mixed formulation (%) | Theoretical mortality (%) | Synergistic effect (%) | Target of Action |
|---|---|---|---|---|---|---|---|---|
| 5+ Thiamethox am | 7.5+15 | 1:2 | 50.8 | 46.9 | 89.7 | 73.9 | 15.8 | *Myzus persicae* |
| 5+Acetamiprid | 7.5+15 | 1:2 | 50.8 | 35.8 | 85.6 | 68.4 | 17.2 | *Myzus persicae* |
| 5+Dinotefuran | 7.5+45 | 1:6 | 50.8 | 62.3 | 98.2 | 81.5 | 16.7 | *Myzus persicae* |
| 5+Clothianidin | 7.5+30 | 1:4 | 50.8 | 52.3 | 93.8 | 76.5 | 17.3 | *Myzus persicae* |
| 5+Sulfoxaflor | 7.5+15 | 1:2 | 50.8 | 39.8 | 91.8 | 70.4 | 21.4 | *Myzus persicae* |
| 5+Flupyradifur one | 7.5+60 | 1:8 | 50.8 | 33.8 | 88.3 | 67.4 | 20.9 | *Myzus persicae* |
| 5+Thiacloprid | 7.5+45 | 1:6 | 50.8 | 62.2 | 92.6 | 81.4 | 11.2 | *Myzus persicae* |
| 5+Triflumezopy rim | 7.5+30 | 1:4 | 50.8 | 45.9 | 92.7 | 73.4 | 19.3 | *Myzus persicae* |
| 5+paichongdin 9 | 7.5+15 | 1:2 | 50.8 | 51.9 | 96.5 | 76.3 | 20.2 | *Myzus persicae* |
| 5+Abamectin | 7.5+3 | 2.5:1 | 50.8 | 33.8 | 85.5 | 67.4 | 18.1 | *Myzus persicae* |
| 5+Emamectin benzoate | 7.5+4.5 | 5:3 | 50.8 | 42.5 | 92.3 | 71.7 | 20.6 | *Myzus persicae* |
| 5+Pyriproxyfen | 30+45 | 1:1.5 | 61.3 | 32.3 | 92.3 | 73.8 | 18.5 | *Bemisia tabaci* |
| 5+Cartap | 30+450 | 1:15 | 61.3 | 35.8 | 90.1 | 75.2 | 14.9 | *Bemisia tabaci* |
| 5+Buprofezin | 30+75 | 1:2.5 | 61.3 | 56.5 | 98.7 | 83.2 | 15.5 | *Bemisia tabaci* |
| 5+Spiromesife n | 30+15 | 2:1 | 61.3 | 30.5 | 89.7 | 73.1 | 16.6 | *Bemisia tabaci* |
| 5+Spirotetrama t | 7.5+45 | 1:6 | 50.8 | 62.3 | 98.8 | 81.5 | 17.3 | *Myzus persicae* |

(continued)

| A+B | A+B Dosage (g a.i./ha) | A:B | Mortality by individual A (%) | Mortality by individual B (%) | Mortality by mixed formulation (%) | Theoretical mortality (%) | Synergistic effect (%) | Target of Action |
|---|---|---|---|---|---|---|---|---|
| 5+Spiropidion | 7.5+30 | 1:4 | 50.8 | 43.2 | 91.9 | 72.1 | 19.8 | *Myzus persicae* |
| 5+Cyantraniliprole | 7.5+30 | 1:4 | 50.8 | 38.5 | 88.6 | 69.7 | 18.9 | *Myzus persicae* |
| 5+Flonicamid | 7.5+30 | 1:4 | 50.8 | 58.8 | 95.9 | 79.7 | 16.2 | *Myzus persicae* |
| 5+Broflanilide | 7.5+30 | 1:4 | 50.8 | 56.2 | 95.9 | 78.5 | 17.4 | *Myzus persicae* |
| 5+Fluxametamide | 7.5+30 | 1:4 | 50.8 | 38.8 | 86.5 | 69.9 | 16.6 | *Myzus persicae* |
| 5+Isocycloseram | 7.5+30 | 1:4 | 50.8 | 48.2 | 93.2 | 74.5 | 18.7 | *Myzus persicae* |
| 5+Compound W | 7.5+15 | 1:2 | 50.8 | 53.2 | 98.2 | 77.0 | 21.2 | *Myzus persicae* |
| 5+Dimpropyridaz | 7.5+7.5 | 1:1 | 50.8 | 49.7 | 89.6 | 75.3 | 14.3 | *Myzus persicae* |
| 5+*Metarhizium anisopliae* | 30 g a.i./ha+30 trillion spores/ha | | 61.3 | 64.9 | 98.6 | 86.4 | 12.2 | *Bemisia tabaci* |
| 5+ Spinosad | 7.5+600 | 1:80 | 50.8 | 0.0 | 60.0 | 50.8 | 9.2 | *Myzus persicae* |

Meanwhile, it has been found by extensive experimentations that the compounds of the present invention and compositions thereof have good control effects on sucking pests (e.g., aphids, whiteflies, stink bugs, woodlice, scale insects, mealybugs, leafhoppers, etc.) and are able to reduce the occurrence of viral and bacterial diseases transmitted by insect vectors. They are suitable for economic crops, field crops, ornamental plants and the like, and can be used for foliage treatment as well as seed treatment or soil treatment. Also, they have little impact on beneficial arthropods such as natural enemy insects and pollinating insects, can effectively lighten the environmental burden of sustainable development in agriculture, and are suitable for pest resistance management and integrated management of harmful organisms, which have certain commercial values.

## Claims

**1.** A pyripyropene derivative as shown in Formula I,

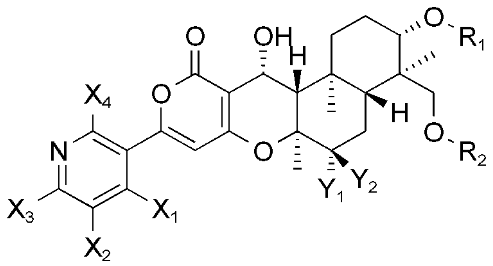

I

wherein, $X_1$ represents hydrogen or fluorine; $X_2$ represents hydrogen or fluorine; $X_3$ represents hydrogen or fluorine; $X_4$ represents hydrogen or fluorine; and $X_1$, $X_2$, $X_3$ and $X_4$ are not simultaneously hydrogen;

$R_1$ and $R_2$ each independently represent hydrogen, alkyl, alkenyl, alkynyl, cycloalkyl, cycloalkylalkyl or -(CO)$R_3$;

$Y_1$ represents hydroxyl, alkoxyalkyloxy, trialkylsilyloxy, or -O(CO)$R_3$; $Y_2$ represents hydrogen or alkyl; or $Y_1$ and $Y_2$ together form =O;

$R_3$ each independently represents hydrogen; alkyl, alkenyl or alkynyl that is unsubstituted or substituted by at least one group selected from halogen, alkoxy, alkoxycarbonyl and alkylthio; cycloalkyl; cycloalkenyl; aryl; or heterocyclyl;

the aforementioned "cycloalkyl", "cycloalkenyl", "heterocyclyl" or "aryl" is optionally substituted by at least one group selected from oxo, halogen, cyano, nitro, alkyl, alkenyl, alkynyl, cycloalkyl, haloalkyl, haloalkenyl, haloalkynyl, halocycloalkyl, alkyl-substituted cycloalkyl, -$OR_{10}$, -$SR_{10}$, -(CO)$OR_{10}$, -($SO_2$)$R_{10}$, - N($R_{10}$)$_2$ and -O-alkylene-(CO)$OR_{10}$, or two adjacent carbon atoms on the ring form a fused ring with unsubstituted or halogen-substituted -$OCH_2CH_2$- or -$OCH_2O$-;

$R_{10}$ each independently represents hydrogen, alkyl, haloalkyl, phenyl, or phenyl that is substituted by at least one group selected from halogen, cyano, nitro, alkyl, haloalkyl, alkoxycarbonyl, alkylthio, alkylsulfonyl, alkoxy and haloalkoxy.

**2.** The pyripyropene derivative according to claim 1, wherein it is **characterized in that**,

$R_1$ and $R_2$ each independently represent hydrogen, C1-C8 alkyl, C2-C8 alkenyl, C2-C8 alkynyl, C3-C8 cycloalkyl, C3-C8 cycloalkyl C1-C8 alkyl or -(CO)$R_3$;

$Y_1$ represents hydroxyl, C1-C8 alkoxy C1-C8 alkyloxy, tri C1-C8 alkylsilyloxy, or -O(CO)$R_3$; $Y_2$ represents hydrogen or C1-C8 alkyl; or $Y_1$ and $Y_2$ together form =O;

$R_3$ each independently represents hydrogen; C1-C8 alkyl, C2-C8 alkenyl or C2-C8 alkynyl that is unsubstituted or substituted by at least one group selected from halogen, C1-C8 alkoxy, C1-C8 alkoxycarbonyl and C1-C8 alkylthio; C3-C8 cycloalkyl; C3-C8 cycloalkenyl; aryl; or heterocyclyl;

the aforementioned "C3-C8 cycloalkyl", "C3-C8 cycloalkenyl", "heterocyclyl" or "aryl" is optionally substituted by at least one group selected from oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C1-C8 alkyl-substituted C3-C8 cycloalkyl, -$OR_{10}$, -$SR_{10}$, -(CO)$OR_{10}$, -($SO_2$)$R_{10}$, -N($R_{10}$)$_2$ and -O-(C1-C8 alkylene)-(CO) $OR_{10}$, or two adjacent carbon atoms on the ring form a fused ring with unsubstituted or halogen-substituted -$OCH_2CH_2$- or -$OCH_2O$-;

$R_{10}$ each independently represents hydrogen, C1-C8 alkyl, halo C1-C8 alkyl, phenyl, or phenyl that is substituted by at least one group selected from halogen, cyano, nitro, C1-C8 alkyl, halo C1-C8 alkyl, C1-C8 alkoxycarbonyl, C1-C8 alkylthio, C1-C8 alkylsulfonyl, C1-C8 alkoxy and halo C1-C8 alkoxy.

**3.** The pyripyropene derivative according to claim 1 or 2, wherein it is **characterized in that**,

$R_1$ and $R_2$ each independently represent hydrogen, C1-C6 alkyl, C2-C6 alkenyl, C2-C6 alkynyl, C3-C6 cycloalkyl, C3-C6 cycloalkyl C1-C6 alkyl or -(CO)$R_3$;

$Y_1$ represents hydroxyl, C1-C6 alkoxy C1-C6 alkyloxy, tri C1-C6 alkylsilyloxy, or -O(CO)$R_3$; $Y_2$ represents hydrogen or C1-C6 alkyl; or $Y_1$ and $Y_2$ together form =O;

$R_3$ each independently represents hydrogen; C1-C6 alkyl, C2-C6 alkenyl or C2-C6 alkynyl that is unsubstituted or substituted by at least one group selected from halogen, C1-C6 alkoxy, C1-C6 alkoxycarbonyl and C1-C6 alkylthio; C3-C6 cycloalkyl; C3-C6 cycloalkenyl; aryl; or heterocyclyl;

the aforementioned "C3-C6 cycloalkyl", "C3-C6 cycloalkenyl", "heterocyclyl" or "aryl" is optionally substituted by 1-3 groups selected from oxo, halogen, cyano, nitro, C1-C6 alkyl, C2-C6 alkenyl, C2-C6 alkynyl, C3-C6

cycloalkyl, halo C1-C6 alkyl, halo C2-C6 alkenyl, halo C2-C6 alkynyl, halo C3-C6 cycloalkyl, C1-C6 alkyl-substituted C3-C6 cycloalkyl, $-OR_{10}$, $-SR_{10}$, $-(CO)OR_{10}$, $-(SO_2)R_{10}$, $-N(R_{10})_2$ and -O-(C1-C6 alkylene)-(CO)$OR_{10}$, or two adjacent carbon atoms on the ring form a fused ring with unsubstituted or halogen-substituted $-OCH_2CH_2-$ or $-OCH_2O-$;

$R_{10}$ each independently represents hydrogen, C1-C6 alkyl, halo C1-C6 alkyl, phenyl, or phenyl that is substituted by 1-3 groups selected from halogen, cyano, nitro, C1-C6 alkyl, halo C1-C6 alkyl, C1-C6 alkoxycarbonyl, C1-C6 alkylthio, C1-C6 alkylsulfonyl, C1-C6 alkoxy and halo C1-C6 alkoxy.

4. The pyripyropene derivative according to any one of claims 1 to 3, wherein it is **characterized in that**, $X_1$ represents hydrogen; $X_2$ represents fluorine; $X_3$ represents hydrogen; $X_4$ represents hydrogen; or, $X_1$ represents hydrogen; $X_2$ represents hydrogen; $X_3$ represents fluorine; $X_4$ represents hydrogen;
preferably, the compound is selected from Table 1 in the Specification.

5. A preparation method of the pyripyropene derivative according to any one of claims 1 to 4, wherein it is **characterized in that**, the pyripyropene derivative is prepared by using

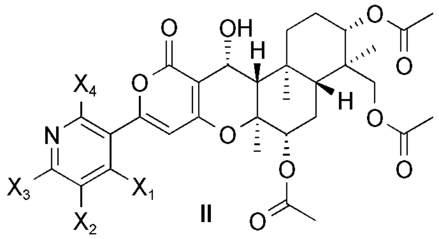

II

as an intermediate; or the pyripyropene derivative is prepared through reduction reaction of

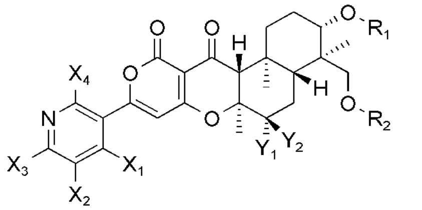

III ;

or, it comprises at least one step of the following steps:

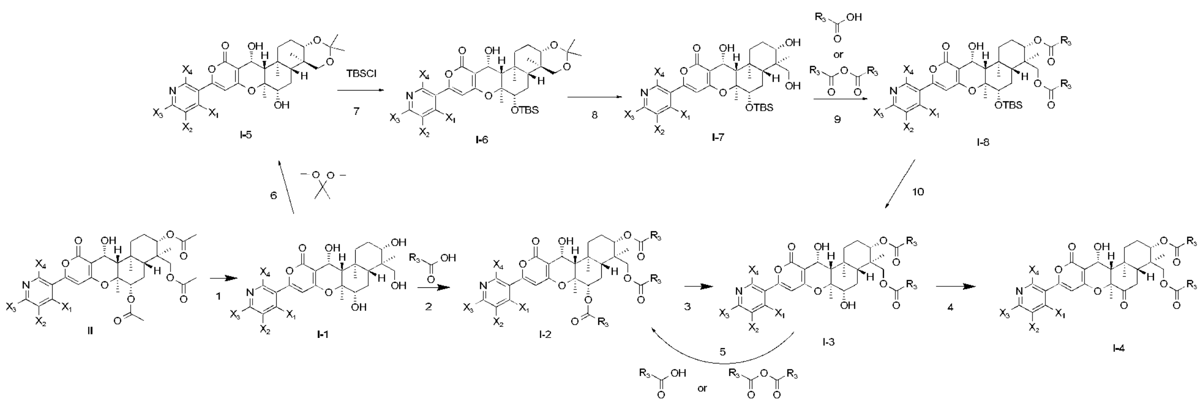


wherein, the definitions of $X_1$, $X_2$, $X_3$, $X_4$ and $R_3$ are as described in any one of claims 1 to 4;
wherein, the reactions in steps 1 and 3 are both carried out in the presence of a solvent and a base;
the reactions in steps 2, 5, and 9 are carried out in the presence of a solvent; preferably, a condensing agent is added during the reactions in steps 2, 5, and 9, with or without the addition of a base; the reaction in step 4 is carried out in the presence of an oxidizing agent and a solvent; the reaction in step 6 is carried out in the presence of an acid catalyst and a solvent; the reaction in step 7 is carried out in the presence of an organic base and a

solvent; the reaction in step 8 is carried out in the presence of an acid (e.g., acetic acid) and a solvent; the reaction in step 10 is carried out in the presence of a pyridine hydrofluoride and a solvent;

more preferably, the solvent is selected from at least one of water, THF, pyridine, DMF, DMA, methanol, ethanol, acetonitrile, dichloroethane, DMSO, dioxane, dichloromethane and ethyl acetate; the base is selected from at least one of inorganic bases and organic bases; the condensing agent is selected from at least one of Py-BOP, Py-AOP, HOBT, EDCI, DMAP, DCC, HBTU and HATU; the oxidizing agent is Dess-Martin periodinane or PCC (pyridinium chlorochromate) oxidizing agent; or the acid catalyst is pyridinium p-toluenesulfonate (PPTS) or p-toluenesulfonic acid;

preferably, the reduction reaction is carried out in the presence of sodium borohydride and a solvent, more preferably, cerium trichloride may be added; or the solvent is selected from at least one of THF, DMF, DMA, methanol, ethanol, acetonitrile, dichloroethane, DMSO, dioxane, dichloromethane, and ethyl acetate.

**6.** The method according to claim 5, wherein it is **characterized in that**, the Compound II is prepared by fermentation culture using *Neosartorya pseudofischeri* with 5-fluoronicotinic acid as a substrate.

**7.** An insecticidal composition, wherein it is **characterized in that**, it comprises a biologically effective amount of at least one of the pyripyropene derivatives according to any one of claims 1 to 4; preferably, it also comprises a formulation adjuvant; more preferably, it also comprises an additional active ingredient; further preferably, the additional active ingredient is selected from at least one of the following compounds:

(1) Acetylcholinesterase (AChE) inhibitors: chlorpyrifos, acephate;
(2) γ-Aminobutyric acid (GABA)-gated chloride channel antagonist: fipronil;
(3) Sodium channel modulators: lambda-cyhalothrin, bifenthrin, deltamethrin, beta-cypermethrin, alpha-cypermethrin;
(4) Nicotinic acetylcholine receptor (nAChR) competitive modulators: imidacloprid, thiamethoxam, acetamiprid, dinotefuran, clothianidin, sulfoxaflor, flupyradifurone, thiacloprid, triflumezopyrim, paichongding;
(5) Glutamate-gated chloride channel (GluCl) allosteric modulators: abamectin, emamectin benzoate;
(6) Juvenile hormone mimics: pyriproxyfen;
(7) Nicotinic acetylcholine receptor (nAChR) channel blockers: cartap;
(8) Inhibitors of chitin biosynthesis, type 1: buprofezin, spiromesifen;
(9) Inhibitors of acetyl CoA carboxylase: spirotetramat, spiropidion;
(10) Ryanodine receptor modulators: cyantraniliprole;
(11) Chordotonal organ modulators - undefined target site: flonicamid, dimpropyridaz;
(12) γ-Aminobutyric acid (GABA)-gated chloride channel allosteric modulators: broflanilide, fluxametamide, isocycloseram, compound W;
(13) Nicotinic acetylcholine receptor (nAChR) allosteric modulators: spinosad;
(14) Others: *Metarhizium anisopliae;*

even further preferably, the weight ratio of the pyripyropene derivative to the additional active ingredient in the insecticidal composition is 1:150-150:1, 1:100-100:1, 1:80-80:1, 1:60-60:1, 1:40-40:1, 1:20-20:1, 1:10-10:1, 1:8-8:1, 1:5-5:1, or 1:3-1:1.

**8.** A method for controlling an insect pest, wherein it is **characterized in that**, it comprises exposing the insect pest or its environment to a biologically effective amount of the pyripyropene derivative according to any one of claims 1 to 4 or the composition according to claim 7.

**9.** Use of the pyripyropene derivative according to any one of claims 1 to 4 or the composition according to claim 7 in controlling harmful organisms.

**10.** An intermediate, as shown in Formula II or **III** according to claim 5.

## INTERNATIONAL SEARCH REPORT

International application No.
**PCT/CN2024/118653**

### A. CLASSIFICATION OF SUBJECT MATTER

C07D493/04(2006.01)i; A01N25/02(2006.01)i; A01N53/00(2006.01)i; A01N53/04(2006.01)i; A01P7/00(2006.01)i; A01P7/02(2006.01)i; A01P7/04(2006.01)i

According to International Patent Classification (IPC) or to both national classification and IPC

### B. FIELDS SEARCHED

Minimum documentation searched (classification system followed by classification symbols)

IPC: C07D A01N A01P

Documentation searched other than minimum documentation to the extent that such documents are included in the fields searched

Electronic data base consulted during the international search (name of data base and, where practicable, search terms used)

CNTXT; VCN; VEN; CNABS; DWPI; WPABS; ENTXT; USTXT; EPTXT; WOTXT; STN; CNKI; ISI-Web of Science; 超星读秀, DUXIU; 万方, WANFANG: 啶南平, 杀虫, 昆虫, 防治, 灭杀, 吡啶, 环酮, pest, insect, pyripyropene, Pyridapine, Insecticide, Control, kill, Pyridine, Cyclic ketone, 式I, 式II, 式III, 式I-5, 式I-6

### C. DOCUMENTS CONSIDERED TO BE RELEVANT

| Category* | Citation of document, with indication, where appropriate, of the relevant passages | Relevant to claim No. |
|---|---|---|
| A | CN 103025161 A (MEIJI SEIKA PHARMA CO., LTD.) 03 April 2013 (2013-04-03) description, paragraph 0020, paragraph 0078, table 31, paragraph 0080, and paragraphs 0091-0117 | 1-10 |
| A | CN 115052598 A (THE KITASATO INSTITUTE et al.) 13 September 2022 (2022-09-13) entire document | 1-10 |
| A | CN 101902916 A (MEIJI SEIKA KAISHA, LTD.) 01 December 2010 (2010-12-01) entire document | 1-10 |
| A | CN 102791871 A (MEIJI SEIKA PHARMA CO., LTD.) 21 November 2012 (2012-11-21) entire document | 1-10 |
| A | JP H08269065 A (THE KITASATO INSTITUTE) 15 October 1996 (1996-10-15) entire document | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 December 2024** | **12 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No. PCT/CN2024/118653

**C. DOCUMENTS CONSIDERED TO BE RELEVANT**

| Category* | Citation of document, with indication, where appropriate, of the relevant passages | Relevant to claim No. |
|---|---|---|
| A | Goto, Kimihiko et al. "Synthesis of Pyripyropene Derivatives and Their Pest-control Efficacy" *Journal of Pesticide Science,* Vol. 44, No. 4, 01 November 2019 (2019-11-01), pages 255-263 ISSN: 1348-589X, Materials and Methods, Fig.1., Scheme 1-2., Results, Discussion | 1-10 |
| A | Goto, Kimihiko et al. "Synthesis and Insecticidal Efficacy of Pyripyropene Derivatives. Part II-Invention of Afidopyropen" *Journal of Antibiotics,* Vol. 72, No. 9, 20 June 2019 (2019-06-20), pages 661-681 ISSN: 0021-8820, Results and Discussion, Fig. 1-5., Scheme 1-8., Table 1-7., Experimental Procedure General Methods | 1-10 |
| A | Obata, Rika et al. "Chemical Modification and Structure-activity Relationships of Pyripyropenes. 3. Synthetic Conversion of Pyridine-pyrone Moiety" *Journal of Antibiotics,* Vol. 50, No. 3, 01 March 1997 (1997-03-01), pages 229-236 ISSN: 0021-8820, Scheme 1-5., Table 1., Experimental | 1-10 |

Form PCT/ISA/210 (second sheet) (July 2022)

**INTERNATIONAL SEARCH REPORT**
**Information on patent family members**

International application No.
**PCT/CN2024/118653**

| Patent document cited in search report | Publication date (day/month/year) | Patent family member(s) | Publication date (day/month/year) |
|---|---|---|---|
| CN 103025161 A | 03 April 2013 | WO 2011148886 A1 | 01 December 2011 |
| | | BR 112012028760 A2 | 06 February 2018 |
| | | JPWO 2011148886 A1 | 25 July 2013 |
| | | AR 082268 A1 | 28 November 2012 |
| | | EP 2578084 A1 | 10 April 2013 |
| | | EP 2578084 A4 | 23 April 2014 |
| | | US 2013131091 A1 | 23 May 2013 |
| | | IN 201211127 P1 | 24 October 2014 |
| | | BR 112012028760 A2 | 06 February 2018 |
| CN 115052598 A | 13 September 2022 | US 2023100006 A1 | 30 March 2023 |
| | | EP 4098259 A1 | 07 December 2022 |
| | | EP 4098259 A4 | 06 March 2024 |
| | | WO 2021153570 A1 | 05 August 2021 |
| | | JP 7430869 B2 | 14 February 2024 |
| | | CA 3169605 A1 | 05 August 2021 |
| CN 101902916 A | 01 December 2010 | TW 200936047 A | 01 September 2009 |
| | | BRPI 0821637 A2 | 14 October 2014 |
| | | RU 2010130535 A | 27 January 2012 |
| | | RU 2492649 C2 | 20 September 2013 |
| | | US 2010281584 A1 | 04 November 2010 |
| | | US 9434739 B2 | 06 September 2016 |
| | | KR 20100094550 A | 26 August 2010 |
| | | NZ 586197 A | 29 June 2012 |
| | | IL 206305 A0 | 30 December 2010 |
| | | IL 206305 A | 31 August 2016 |
| | | WO 2009081851 A1 | 02 July 2009 |
| | | EP 2223599 A1 | 01 September 2010 |
| | | EP 2223599 A4 | 11 January 2012 |
| | | JPWO 2009081851 A1 | 06 May 2011 |
| | | JP 5649824 B2 | 07 January 2015 |
| | | CA 2709466 A1 | 02 July 2009 |
| | | AU 2008342070 A1 | 02 July 2009 |
| | | AU 2008342070 B2 | 31 July 2014 |
| | | MX 2010006603 A1 | 31 July 2010 |
| | | VA 24455 A | 25 November 2010 |
| | | IN 201004393 P1 | 26 November 2010 |
| | | ZA 201004472 A | 28 September 2011 |
| | | CN 101902916 B | 16 April 2014 |
| | | MX 322878 B | 19 August 2014 |
| CN 102791871 A | 21 November 2012 | JPWO 2011093187 A1 | 06 June 2013 |
| | | AU 2011210969 A1 | 06 September 2012 |
| | | CA 2787829 A1 | 04 August 2011 |
| | | RU 2012136447 A | 10 March 2014 |
| | | BR 112012019457 A2 | 15 September 2015 |
| | | WO 2011093187 A1 | 04 August 2011 |
| | | KR 20120127617 A | 22 November 2012 |
| | | MX 2012008688 A | 23 August 2012 |
| | | EP 2530165 A1 | 05 December 2012 |
| | | EP 2530165 A4 | 25 December 2013 |
| | | US 2012330022 A1 | 27 December 2012 |

Form PCT/ISA/210 (patent family annex) (July 2022)

**INTERNATIONAL SEARCH REPORT**
**Information on patent family members**

International application No.
**PCT/CN2024/118653**

| Patent document cited in search report | Publication date (day/month/year) | Patent family member(s) | Publication date (day/month/year) |
|---|---|---|---|
| | | IL 221130 A | 24 September 2012 |
| | | VN 32959 A | 25 March 2013 |
| | | IN 201207303 P1 | 18 December 2015 |
| JP H08269065 A | 15 October 1996 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6222100 B
- WO 0182685 A
- WO 0026390 A
- WO 9741218 A
- WO 9802526 A
- WO 9802527 A
- WO 04106529 A
- WO 0520673 A
- WO 0314357 A
- WO 0313225 A
- WO 0314356 A
- WO 0416073 A
- WO 9200377 A
- EP 0242236 A
- EP 242246 A
- US 5559024 A
- WO 02015701 A
- EP 374753 A
- WO 93007278 A
- WO 9534656 A
- EP 427529 A
- EP 451878 A
- WO 03018810 A
- WO 03052073 A
- EP 039222 A
- US 4822779 A
- US 6300348 B
- WO 200872743 A
- WO 200872783 A
- WO 2007043677 A
- WO 2007101540 A
- JP 2006131529 B
- WO 2007060839 A
- WO 2005085216 A
- WO 2007079162 A
- WO 2007026965 A
- WO 2009126668 A
- WO 2009051956 A
- WO 2007115644 A
- WO 200866153 A
- WO 2008108491 A
- JP 2008115155 A
- WO 02089579 A
- WO 02090320 A
- WO 02090321 A
- WO 04006677 A
- WO 05068423 A
- WO 05068432 A
- WO 05063694 A
- WO 2011093186 A
- WO 2010010955 A
- WO 2011148886 A


### Non-patent literature cited in the description


- *Biotechnol Prog.*, July 2001, vol. 17 (4), 720-8
- *Protein Eng Des Sel.*, January 2004, vol. 17 (1), 57-66
- *Nat. Protoc.*, 2007, vol. 2 (5), 1225-35
- *Curr Opin Chem. Biol.*, October 2006, vol. 10 (5), 487-91
- *Biomaterials*, March 2001, vol. 22 (5), 405-17
- *Bioconjug Chem.*, January 2005, vol. 16 (1), 113-21
- The Pesticide Manual. he British Crop Protection Council
- SHIBUYA INDEX. SHIBUYA INDEX RESEARCH GROUP, 2009
- Farm Chemicals Handbook. Meister Publishing Company, 2001, vol. 88
- *Pesticide Science*, 1988, vol. 54, 237-243
- *CHEMICAL ABSTRACTS*, 2921-88-2
- *CHEMICAL ABSTRACTS*, 30560-19-1
- *CHEMICAL ABSTRACTS*, 120068-37-3
- *CHEMICAL ABSTRACTS*, 91465-08-6
- *CHEMICAL ABSTRACTS*, 82657-04-3
- *CHEMICAL ABSTRACTS*, 52918-63-5
- *CHEMICAL ABSTRACTS*, 1224510-29-5
- *CHEMICAL ABSTRACTS*, 67375-30-8
- *CHEMICAL ABSTRACTS*, 138261-41-3
- *CHEMICAL ABSTRACTS*, 153719-23-4
- *CHEMICAL ABSTRACTS*, 135410-20-7
- *CHEMICAL ABSTRACTS*, 165252-70-0
- *CHEMICAL ABSTRACTS*, 210880-92-5
- *CHEMICAL ABSTRACTS*, 946578-00-3
- *CHEMICAL ABSTRACTS*, 951659-40-8
- *CHEMICAL ABSTRACTS*, 111988-49-9
- *CHEMICAL ABSTRACTS*, 1263133-33-0
- *CHEMICAL ABSTRACTS*, 948994-16-9
- *CHEMICAL ABSTRACTS*, 71751-41-2
- *CHEMICAL ABSTRACTS*, 155569-91-8
- *CHEMICAL ABSTRACTS*, 95737-68-1
- *CHEMICAL ABSTRACTS*, 15263-53-3

- *CHEMICAL ABSTRACTS*, 69327-76-0
- *CHEMICAL ABSTRACTS*, 283594-90-1
- *CHEMICAL ABSTRACTS*, 203313-25-1
- *CHEMICAL ABSTRACTS*, 1229023-00-0
- *CHEMICAL ABSTRACTS*, 736994-63-1
- *CHEMICAL ABSTRACTS*, 158062-67-0
- *CHEMICAL ABSTRACTS*, 1403615-77-9
- *CHEMICAL ABSTRACTS*, 1207727-04-5
- *CHEMICAL ABSTRACTS*, 928783-29-3
- *CHEMICAL ABSTRACTS*, 2061933-85-3
- *CHEMICAL ABSTRACTS*, 2892524-05-7
- *CHEMICAL ABSTRACTS*, 168316-95-8